# EUROPEAN PATENT SPECIFICATION

(11) **EP 2 419 512 B1**
(45) Date of publication and mention of the grant of the patent: **28.08.2013**
(21) Application number: 10764280.3
(22) Date of filing: 15.04.2010
(51) Int. Cl.: C12N 15/09, C07K 14/47, C07K 19/00, C12N 5/10, C12Q 1/02, G01N 33/15, G01N 33/50

(54) **METHOD OF SCREENING FOR INSULIN SECRETION-POTENTIATING AGENTS**
VERFAHREN ZUM SCREENING NACH INSULINAUSSCHEIDUNGSPOTENZIERENDEN MITTELN
PROCÉDÉ DE CRIBLAGE D'AGENTS DE POTENTIALISATION DE LA SÉCRÉTION D'INSULINE

(30) Priority: 17.04.2009 JP 2009100794
(43) Date of publication of application: 22.02.2012
(73) Proprietor: Kobe University, Hyogo 657-0013 (JP); JCR Pharmaceuticals CO., LTD., Ashiya-shi, Hyogo 659-0021 (JP)
(72) Inventor: SEINO, Susumu, Kobe-shi Hyogo 657-0013 (JP); ZHANG, Chang-Liang, Kobe-shi Hyogo 657-0013 (JP); KATO, Megumi, Kobe-shi Hyogo 657-0013 (JP); SHIBAZAKI, Tadao, Kobe-shi Hyogo 657-0013 (JP)
(74) Representative: Isarpatent
(86) International application number: PCT/JP2010/002755
(87) International publication number: WO 2010/119693

(56) References cited:
- WO-A1-2005/052186
- WO-A1-2005/052186
- WO-A2-2006/023972
- WO-A2-2006/054167
- C.-L. ZHANG ET AL: "The cAMP Sensor Epac2 Is a Direct Target of Antidiabetic Sulfonylurea Drugs", SCIENCE, vol. 325, no. 5940, 30 July 2009 (2009-07-30), pages 607-610, XP55038924, ISSN: 0036-8075, DOI: 10.1126/science.1172256
- , 1 January 2006 (2006-01-01), XP55038883, Retrieved from the Internet: URL:http://physiologyonline.physiology.org /content/21/2/86.full.pdf#page=1&view=FitH [retrieved on 2012-09-21]
- DIPILATO, L.M. ET AL.: 'Fluorescent indicators of cAMP and Epac activation reveal differential dynamics of cAMP signaling within discrete subcellular compartments.' PROC. NATL. ACAD. SCI. USA vol. 101, no. 47, 23 November 2004, pages 16513 - 16518
- REHMANN, H. ET AL.: 'Structure of Epac2 in complex with a cyclic AMP analogue and RAP1B.' NATURE vol. 455, no. 7209, 04 September 2008, pages 124 - 127
- SHIBASAKI, T. ET AL.: 'Essential role of Epac2/Rapl signaling in regulation of insulin granule dynamics by cAMP.' PROC. NATL. ACAD. SCI. USA vol. 104, no. 49, 04 December 2007, pages 19333 - 19338
- KANG, G. ET AL.: 'cAMP sensor Epac as a determinant of ATP-sensitive potassium channel activity in human pancreatic beta cells and rat INS-1 cells.' J. PHYSIOL. vol. 573, no. 3, 15 June 2006, pages 595 - 609
- PONSIOEN, B. ET AL.: 'Detecting cAMP-induced Epac activation by fluorescence resonance energy transfer: Epac as a novel cAMP indicator.' EMBO REP. vol. 5, no. 12, December 2004, pages 1176 - 1180
- VAN DER KROGT, G.N. ET AL.: 'A comparison of donor-acceptor pairs for genetically encoded FRET sensors: application to the Epac cAMP sensor as an example.' PLOS ONE vol. 3, no. 4, 02 April 2008, page E1916
- SOMASEKAR SESHAGIRI ET AL: 'Recurrent R-spondin fusions in colon cancer' NATURE vol. 488, no. 7413, 01 January 2012, pages 660 - 664, XP055060432 DOI: 10.1038/nature11282 ISSN: 0028-0836

## Description

### Technical Field

The present invention relates to a method of screening for insulin secretion-potentiating agents, and more specifically to a method of screening for insulin secretion-potentiating agents utilizing fluorescent-labeled Epac2.

### Background Art

Sulfonylureas (hereinafter referred to as "SU agents"), which are widely used as therapeutic agents for diabetes, are known to exhibit their effects through binding to an SU receptor (SUR1), a component and regulatory subunit of ATP-sensitive K⁺ channels (K_{AT}p channels) (Non-patent documents 1-3). K_{AT}p channels occur on the cell membrane of pancreatic beta-cells and regulate through their opening and closing the amount of insulin to be secreted. Binding of an SU agent to the SU receptors induces closure of the K_{AT}p channels on the surface of pancreatic beta-cells, which brings about depolarization of the cell membrane. This then causes voltage-dependent Ca²⁺ channels to open to allow Ca²⁺ influx into the cells, thereby potentiating insulin secretion. Examples of SU agents include tolbutamide, glibenclamide, chlorpropamide, gliclazide, and the like.

Multiple intracellular signals take part in the mechanism for regulation of insulin secretion. In particular, cAMP occurring in the cell functions as a very important signal molecule for regulation of insulin secretion. It is known that within the mechanism for regulation of insulin secretion caused by cAMP, there are two types of pathways, which are protein kinase A-dependent and independent pathways. Among the protein kinase A-independent pathways, there is known a pathway which is mediated by Epac 2 (exchange protein directly activated by cAMP 2: a guanine-nucleotide-exchanging factor (GEF) for the small G-protein Rap1 that is activated by cAMP) (Non-patent documents 4-6). Epac2 activated by direct binding of cAMP has a guanine-nucleotide-exchanging activity for Rap1, and this activity activates Rap1, thereby promoting insulin secretion.

As noted above, while there are a variety of mechanisms for regulation of insulin secretion, the mechanisms of actions of therapeutic agents for diabetes, including SU agents, are yet to be fully elucidated.

There is an isoform of Epac2 structurally similar to it (Epac1) (Non-patent document 7). Epac1 has a cAMP-binding domain, a Dishevelled, Egl-10, Plechstrin (DEP) domain, which plays a role in membrane localization of Epac1, a Ras-exchanger motif (REM), and a GEF domain at the carboxy terminus. Epac2, which is structurally similar to Epac1, has in addition to the above domains, a second cAMP-binding domain at its amino-terminus and a Ras-association (RA) domain that interacts with Ras. Both Epac1 and Epac2 take part in intracellular signal transmission via cAMP.

For monitoring signal transmission via cAMP within the cell, a fusion protein has been developed as fluorescence resonance energy transfer (FRET) sensor which monitors Epac1 activation in living cells, in which protein partial or full-length Epac1 is sandwiched between two other proteins which emit fluorescent light with different colors, i.e., cyan and yellow fluorescent proteins (Non-patent documents 8-10).

"FRET" is a phenomenon that when one (donor) of two dye molecules, e.g., fluorescent proteins, located in close proximity with each other is irradiated with light having a certain wavelength (excitation light) to excite the dye molecule, the excitation energy is transferred to the other dye molecules (acceptor) located in close proximity through resonance between electrons. According to this phenomenon, light having a certain wavelength (fluorescent light) is emitted from the acceptor by the energy of light absorbed by the donor. Thus, the FRET technique is a method to detect changes in fluorescence caused by FRET.

When a fusion protein consisting of cyan and yellow fluorescent proteins and partial or full-length Epac1 sandwiched between them (fluorescent-labeled Epac1) is irradiated with excitation light (wavelength: approx. 440 nm) for the cyan fluorescent protein, (which emits fluorescent light with the shorter wavelength), the protein is excited, and FRET taking place with part of this excitation energy, the yellow fluorescent protein is also excited and emits fluorescent light having the wavelength at 535 nm. On the other hand, part of the excitation energy in the cyan fluorescent protein does not cause FRET, but fluorescent light having the wavelength at 480 nm is emitted from the cyan fluorescent protein. Thus, the ratio in intensity between the fluorescent light having two different wavelengths emitted from the fluorescent-labeled Epac1 (fluorescence intensity at the wavelength of 535 nm/fluorescence intensity at the wavelength of 480 nm) is dependent on the conformation and distance between the cyan and yellow fluorescent proteins in the molecule. Therefore, when the conformation of the molecule changes due to some modification or binding with other molecules, the ratio also changes accordingly. As the high-order structure of fluorescent-labeled Epac1, for example, changes when cAMP binds to the molecule, detection of this change by the FRET technique allows detection of whether a cAMP molecule binds to Epac1 or not. Thus, by measuring the change in the intensity of fluorescent light emitted using the FRET technique while irradiating fluorescent-labeled Epac1 with light having the wavelength at 440 nm, presence of signal transmission via Epac1 can be detected.

While a method has been known to measure signal transmission employing Epac1 as a sensor in the FRET technique (FRET sensor) as mentioned above, no method is known in which full-length Epac2 is utilized as a FRET sensor.

Epac2 is known to mediate insulin secretion via a cAMP-dependent, protein kinase A (PKA)-independent pathway (Non-patent documents 11 and 12). Epac2 was originally identified as an interacting molecule (cAMP-GEF II) with SUR1, a regulatory subunit of K_{ATP} channels (Non-patent document 13).

SUR1 is known to be the target molecule of SU agents. Binding of an SU agent to SUR1 induces the K_{ATP} channel closure on the one hand and on the other hand let the voltage-dependent Ca²⁺ channels open, and this, by allowing Ca²⁺ influx into the beta-cells, enhances insulin secretion (Non-patent document 2). Studies of mice lacking Kir6.2 gene, a subunit of K_{ATP} channels, and mice lacking SUR1 gene confirm that closure of the K_{AT}p channels is prerequisite for SU agents to enhance insulin secretion (Non-patent documents 14-16). Thus, it has been thought that it is via K_{ATP} channels that SU agents exhibit their pharmacological effects as therapeutic agents for diabetes.

Recently, mutations in Kir6.2 or SUR1 have been shown to cause neonatal diabetes mellitus with various symptoms due to the impaired function of K_{ATP} channels (Non-patent document 17). Glycemic control was found to be improved in many of such patients when insulin injection was replaced by oral high-dose SU agents (Non-patent document 18).

Further, there is a clinical report that among SU agents, gliclazide was not effective in a patient with neonatal diabetes, while glibenclamide improved glycemic control in such a patient (Non-patent document 19). The finding suggests that the mechanism of action of SU agents is not uniform. Thus, it is essential to know the mechanisms of action of SU agents for providing proper treatment for patients with neonatal diabetes. Furthermore, elucidation of unknown mechanisms of action is important, for it would provide a clue to and means for development of a new way of medical treatment of diabetic patients with diverse background factors, as well as for development of new therapeutic agents for such treatment.

### Citation List

### Non Patent Literature

NPL 1: Seino S., (1999) Annu. Rev. Physiol. 61, 337-62
NPL 2: Henquin J.C., (2000) Diabetes 49, 1751-60
NPL 3: Proks P. et.al., (2002) Diabetes 51, S368-76
NPL 4: de Rooji J. et.al., (1998) Nature 396, 474-7
NPL 5: Kawasaki H. et.al., (1998) Science 282, 2275-9
NPL 6: de Rooji J. et.al., (2000) J. Biol. Chem. 275, 20829-36
NPL 7: Bos J.L. et.al., (2003) Mol. Cell. Biol. 4, 733-8
NPL 8: Nikolaev V.O. et.al., (2004) J. Biol. Chem. 279, 37215-8
NPL 9: Dipilato L.M. et.al., (2004) Proc. Natl. Acad. Sei. USA. 101, 16513-8
NPL 10: Ponsioen B. et.al., (2004) EMBO Rep. 5, 1176-80
NPL 11: Holz G.G. et.al., (2004) Diabetes 53, 5-13
NPL 12: Seino S. et.al., (2005) Physiol. Rev. 85, 1303-42
NPL 13: Ozaki N. et.al., (2000) Nat. Cell. Biol. 2, 805-811
NPL 14: Miki T. et.al., (1998) Proc. Natl. Acad. Sei. USA. 95, 10402-6
NPL 15: Seghers V. et.al., (2000) J. Biol. Chem. 275, 9270-7[NPL 16] Shiota C. et.al., (2002) J. Biol. Chem. 277, 37176-83
NPL 17: Hattersley A. T. et.al., (2005) Diabetes 54, 2503-13
NPL 18: Pearson E. R. et.al., (2006) N. Engl. J. Med. 355, 467-77
NPL 19: Koster J. C., (2008) J. Clin. Endocrinol. Metab. 93, 1054-61
Chang -Liang Zhang et al. disclose in "The cAMP Sensor Epac2 is a Direct Target of Antidiabetic Sulfonylurea Drugs", Science 325, 607, 2009 that fluorescence resonance energy transfer and binding experiments were used to study the interaction of Epac2 with widely used antidiabetic drugs.
Viacheslav O. Nikolaev and Martin J. Lohse describe in "Monitoring of cAMP Synthesis and Degradation in Living Cells", Physiology 21, 86-92, 2006 the comparison of electrophysiological and fluorescent methods that have been developed based on activation of all three types of cAMP effectors and are used for monitoring and visualizing cAMP in intact living cells.

### Summary of Invention

### Technical Problem

Against the above background, it is an object of the present invention to provide a method of screening for new insulin secretion-potentiating agents which target Epac2, the method utilizing Epac2 fused with two fluorescent proteins as a sensor. It is another object of the present invention to provide a method which is based on their binding to Epac2, of screening of known antidiabetic agents including SU agents, for finding out how they are used properly.

### Solution to Problem

The present inventors found a new mechanism of action of SU agents, which had been known to target the SUR1 molecule, a mechanism in which they act via their binding to Epac2. The present invention was completed on the basis of this finding and through further studies.

Thus the following is described.
1. A DNA encoding fluorescent-labeled Epac2 comprising two different DNAs encoding two different fluorescent proteins which emit fluorescent light with wavelength differing from each other and a DNA encoding Epac2 which are fused together in-frame.
2. The DNA according to 1 above, wherein the two different fluorescent proteins are a cyan fluorescent protein and a yellow fluorescent protein.
3. The DNA according to 2 above, wherein the DNA encoding the cyan fluorescent protein and the DNA encoding the yellow fluorescent protein are fused to the 5'- and 3'-terminuses, respectively, of the DNA encoding Epac2.
4. The DNA according to 2 or 3 above, wherein the cyan fluorescent protein is ECFP and the yellow fluorescent protein is EYFP.
5. An expression vector comprising an incorporated DNA according to one of 1 to 4 above.
6. The expression vector according to 5 above, wherein the expression vector is for mammalian cells.
7. A cell which is a transformant carrying the expression vector according to 5 or 6 above.
8. The cell according to 7 above, wherein the cell is a mammalian cell.
9. The cell according to 8 above, wherein the cell does not express SUR1 gene.
10. The cell according to 9 above which is a COS cell.
11. A fluorescent-labeled Epac2 which is a fusion protein comprising Epac2 and two different fluorescent proteins fused thereto, wherein the two different fluorescent proteins emit fluorescent light with wavelength differing from each other.
12. A fluorescent-labeled Epac2 which is a fusion protein comprising two different fluorescent proteins and Epac2, wherein the fusion protein is expressed in the cell according to one of 7 to 10 above.
13. A method of screening for an insulin secretion-potentiating agent comprising the steps of providing candidate compounds for an insulin secretion-potentiating agent, bringing each candidate compound into contact with the cell according one of 7 to 10 above, irradiating the cell with the excitation light for the shorter wavelength fluorescent protein of the two different fluorescent proteins before and after the contact of
   the cell with the candidate compound to measure the intensity of the fluorescent light emitted from each of the two different fluorescent proteins included in the fluorescent-labeled Epac2 in the cell, detecting changes in the intensity ratio between the two
   different fluorescent lights before versus after the contact of the candidate compound with the cell, and selecting a candidate compound which caused a change as an insulin secretion-potentiating agent.
   The present invention provides what follows.
14. A method of screening for an insulin secretion-potentiating agent comprising the steps of providing candidate compounds for an insulin secretion-potentiating agent, bringing each candidate compound into contact with the fluorescent-labeled Epac2, which is a fusion protein comprising Epac2 and two different fluorescent proteins fused thereto, wherein the two different fluorescent proteins emit fluorescent light with wavelength differing from each other, irradiating the same with the excitation light for the shorter wavelength fluorescent protein of the two different fluorescent proteins before and after the contact of the same with the candidate compound to measure the intensity of the fluorescent light emitted from each of the two different fluorescent proteins included in the fluorescent-labeled Epac2, detecting changes in the intensity ratio between the two different fluorescent lights before versus after the contact with the candidate compound, and selecting a candidate compound which caused a change as an insulin secretion-potentiating agent.

### Advantageous Effects of Invention

The present invention enables to perform screening for insulin secretion-potentiating agents which target Epac2. As no insulin secretion-potentiating agent which targets Epac2 is known so far, the present invention can be used in screening for anti-diabetic agents which work by a novel mechanism of action. Considering that the mechanism of the development of diabetes is not fully understood, and that there are patients who resist current medical treatments, the method according to the present invention is meaningful.

### Brief Description of Drawings

[fig.1]

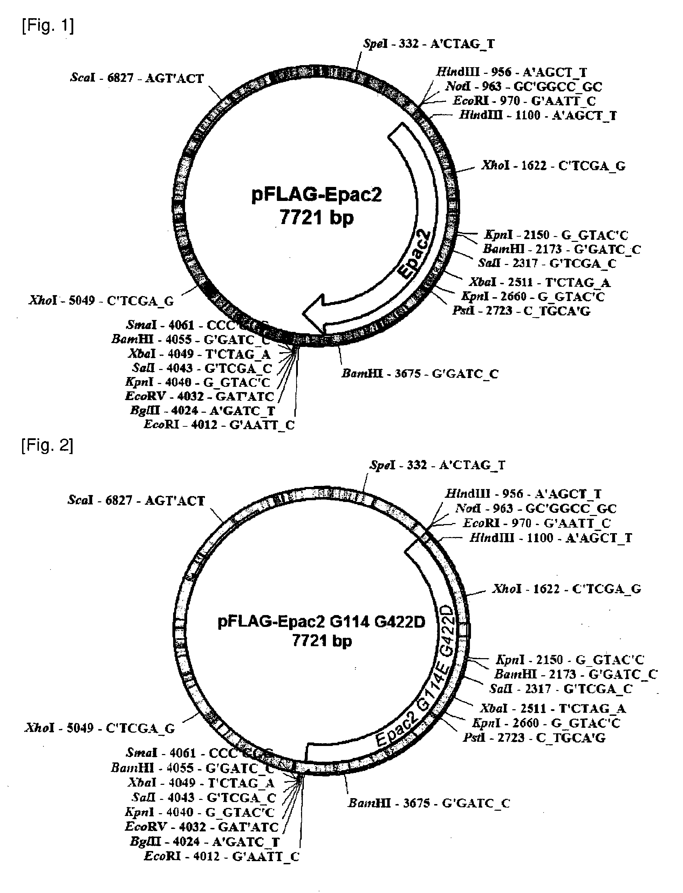

Fig. 1 is a vector map for a wild-type mouse Epac2 expression vector, pFLAG-Epac2.
[fig.2]Fig. 2 is a vector map for a mutant-type mouse Epac2 expression vector, pFLAG-Epac2 G114E G244D.

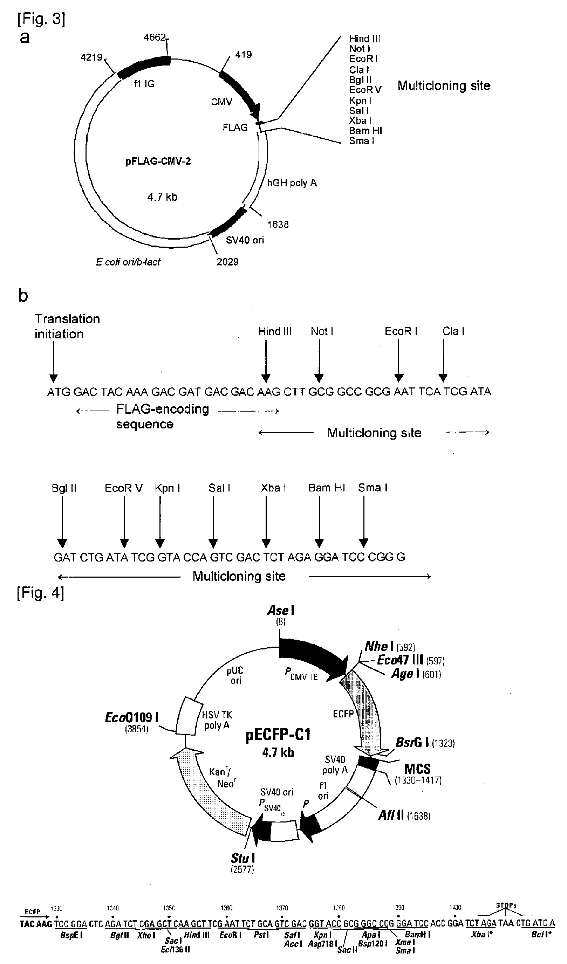

[fig.3]Fig. 3a is a vector map for pFLAT-CMV-2, and Fig. 3b the nucleotide sequence of its multicloning site.
[fig.4]Fig. 4 shows a gene map for vector pECFP-C1 and the nucleotide sequence of its multicloning site.

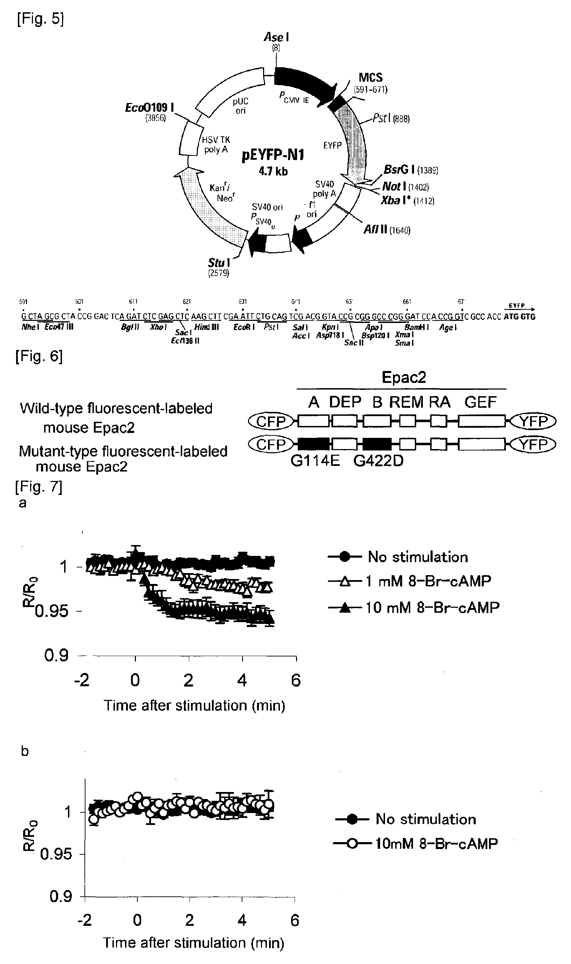

[fig.5]Fig. 5 shows a gene map for vector pEYFP-N1 and the nucleotide sequence of its multicloning site.
[fig.6]Fig. 6 is a schematic diagram of the secondary structures of fluorescent-labeled mouse Epac2 and a fluorescent-labeled, mutant-type mouse Epac2: ECFP: cyan fluorescent protein, EYFP: yellow fluorescent protein, A and B: cAMP-binding domain, DEP: [dishevelled, Egl-10, Plechstrin domain, REM: Ras-exchanger motif, RA: Ras-association domain, GEF: GEF domain.
[fig.7]Fig. 7 is a graph showing the dynamic analysis using the FRET technique of fluorescent-labeled mouse Epac2 in COS-1 cells in the presence of 8-Bromo-cAMP. The vertical axis indicates R/R₀ values, and the horizontal axis the time that has lapsed since the addition of 8-Bromo-cAMP. Data are shown for (a) fluorescent-labeled mouse Epac2, and (b) a fluorescent-labeled, mutant-type mouse Epac2, respectively.

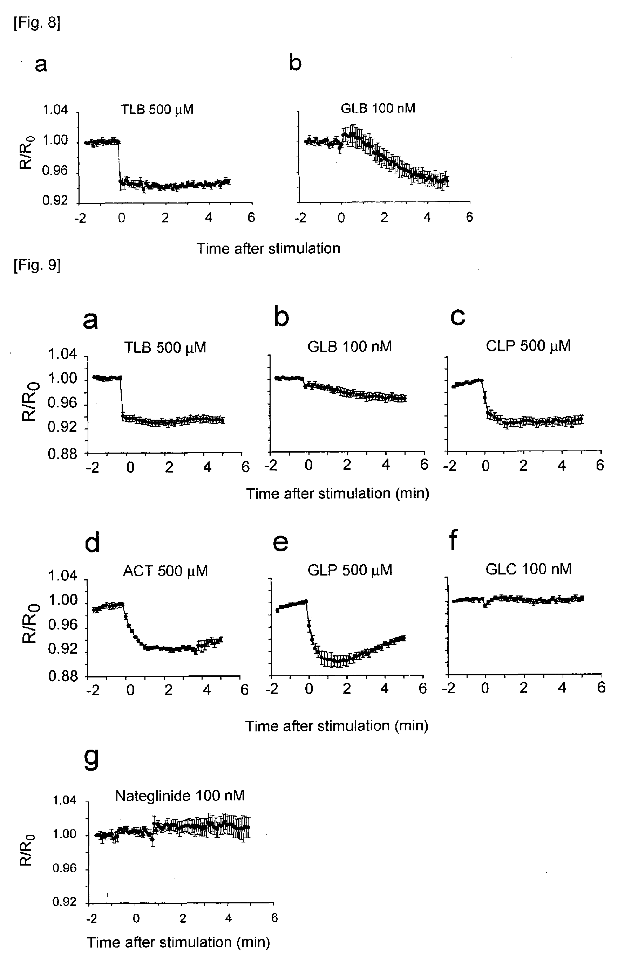

[fig.8]Fig. 8 is a graph showing the effect of SU agents on the dynamic analysis using the FRET technique of fluorescent-labeled mouse Epac2 in MIN6 cells. The vertical axis indicates R/R₀ values, and the horizontal axis the time that has lapsed since the addition of SU agents. As SU agents, (a) tolbutamide and (b) glibenclamide were added, respectively.
[fig.9]Fig. 9 is a graph showing the effect of SU agents on the dynamic analysis of fluorescent-labeled mouse Epac2 in COS-1 cells using the FRET technique. The vertical axis indicates R/R₀ values, and the horizontal axis the time that has lapsed since the addition of SU agents. As SU agents, (a) tolbutamide, (b) glibenclamide, (c) chlorpropamide, (d) acetohexamide, (e) glipizide, (f) gliclazide, and (g) nateglinide were added, separately.

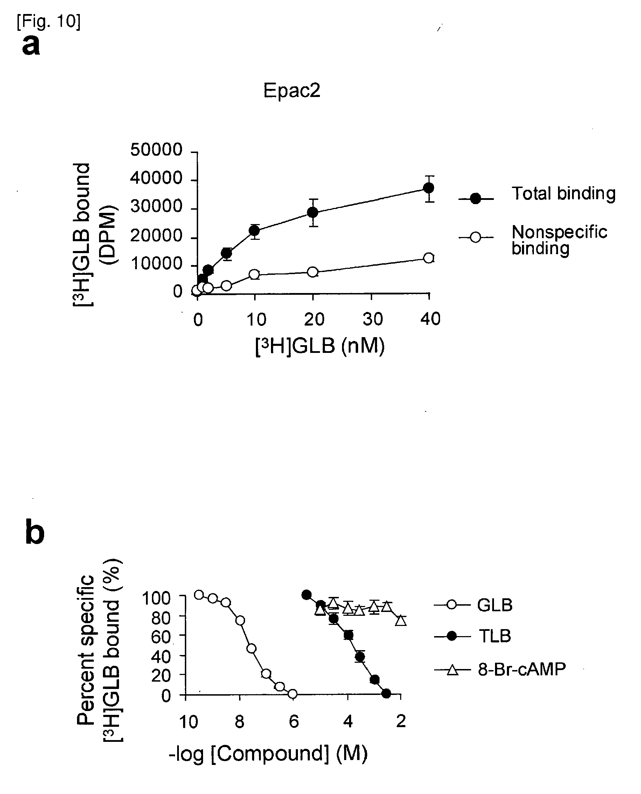

[fig.10] Fig. 10 is a graph showing the competitive property of the biding of tritium-labeled glibenclamide to mouse Epac2. (a) Black circle: Total binding, Open circle: Binding in the presence of 100 micro M unlabeled glibenclamide. The vertical axis indicates the radioactivity (DPM) of bound tritium-labeled glibenclamide, and the horizontal axis the amount of tritium-labeled glibenclamide which was added. (b) Open circle: unlabeled glibenclamide, Black circle: tolbutamide, Open triangle: 8-Bromo-cAMP. The vertical axis indicates the proportion (%) of bound tritium-labeled glibenclamide, and the horizontal axis the amount (-log M) of the compounds added to for competition.

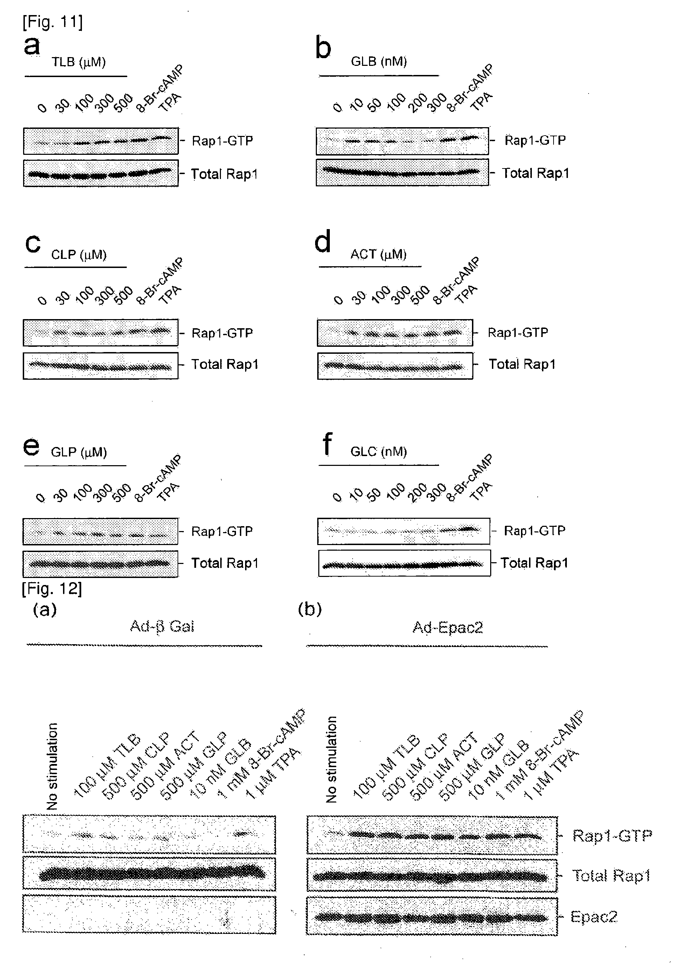

[fig. 11]Fig. 11 shows activation of Rap 1 inducted by SU agents in MIN6 cells. In each figure, the upper part indicates the amount of GTP-bound Rap 1, the lower part the total amount of Rap1. (a) Tolbutamide, (b) glibenclamide, (c) chlorpropamide, (d) acetohexamide, (e) glipizide, and (f) gliclazide were added, respectively.
[fig. 12] Fig. 12 shows activation of Rap1 in Epac2-lacking pancreatic beta-cells by SU agents. Represented by the signs in the figure are: TLB: tolbutamide, CLP: chlorpropamide, ACT: acetohexamide, GLP: glipizide, and GLB: glibenclamide, respectively. The data are from (a) Epac2-lacking pancreatic beta-cells, and (b) Epac2-lacking pancreatic beta-cells in which introduced mouse Epac2 is expressed.

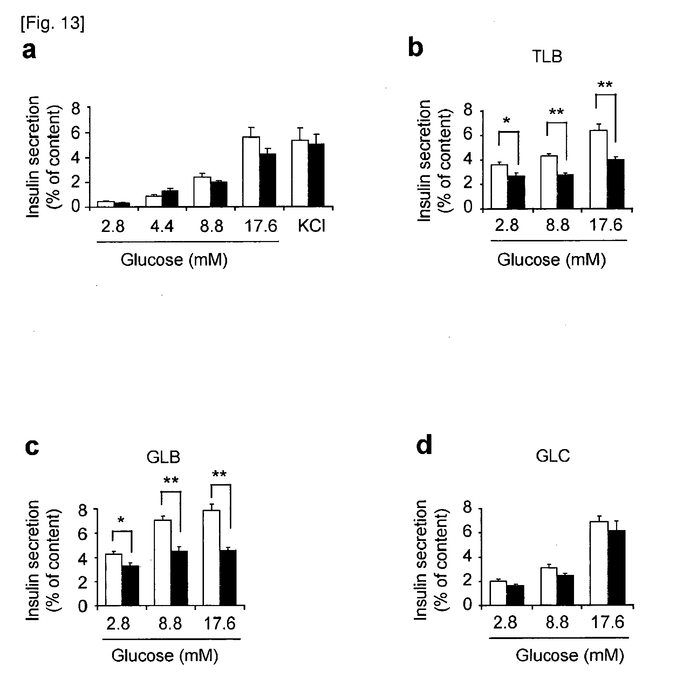

[fig.13]Fig. 13 is a graph showing the amount of insulin secreted from the pancreatic beta-cells of wild-type mice (open bars) and Epac2-lacking mice (black bars), respectively. (a)Glucose or KC1, (b) tolbutamide, (c) glibenclamide, and (d) gliclazide were added, respectively.

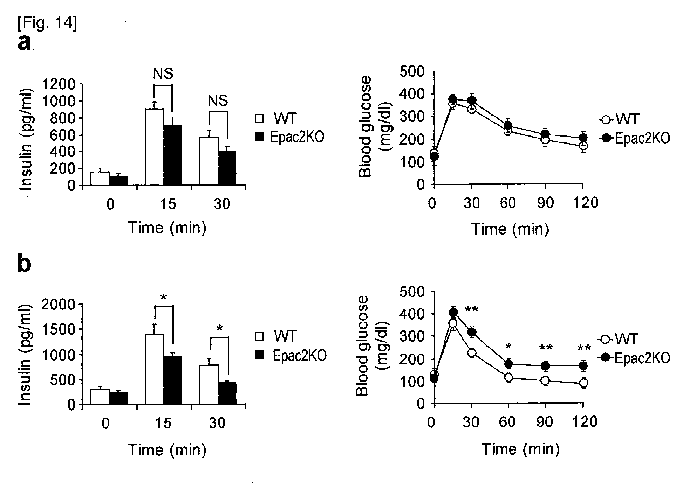

[fig. 14]Fig. 14 is a graph showing serum insulin concentration and blood glucose concentration in wild-type mice (WT) and Epac2-lacking mice (Epac2KO). Open bars and open circles indicate data for wild-type mice, and black bars and black circles for Epac2-lacking mice. (a) Glucose alone was, or (b) glucose and tolbutamide were, administered.

### Description of Embodiments

In the present invention, "Epac2", when simply so referred to, means mammalian Epac2, in particular mouse and human Epac2, and inter alia, wild-type Epac2. The cDNA sequence of mouse wild-type Epac2 is shown as SEQ ID NO:1, and the amino acid sequence as SEQ ID NO:2, respectively. The cDNA sequence of human wild-type Epac2 is shown as SEQ ID NO:3, and the amino acid sequence as SEQ ID NO:4, respectively. Human Epac2 cDNA can be obtained in a publicly known manner (Non-patent document 5).

The amino acid sequences of mouse and human wild-type Epac2 both consist of 1011 amino acids residues, which differ in only 25 amino acids from each other. And in each of five out of those different amino acids, the difference is limited between similar amino acid pairs (one acidic amino acid, one basic amino acid, two branched amino acids, and one hydroxy amino acid). Thus, the homology between them is very high, i.e., not less than 98%. This high homology strongly suggests their substantial equivalence in structure and function. Meanwhile, in the present invention, "Epac2", when simply so referred to, includes not only the naturally occurring full-length Epac2 but also such peptides as comprising a partial amino acid sequence of Epac2 including its cAMP-binding domain, Dishevelled, Egl-10, Plechstrin (DEP) domain, which plays a role in membrane localization, Ras-exchanger motif (REM) domain, GEF domain at the carboxy terminus, cAMP-binding domain at the amino terminus, and Ras-association (RA) domain, which interacts with Ras.

In the present invention, "fluorescent-labeled Epac2" means a protein comprising Epac2 and two different fluorescent proteins which are fused to the former. Although any combination of these two different fluorescent proteins is allowed to be fused to Epac2 insofar as the combination of the fluorescent protein, fused to Epac2, can cause FRET, preferred is a combination of a cyan fluorescent protein and a yellow fluorescent protein, and more preferred is the combination of ECFP (Enhanced Cyan Fluorescence Protein) and EYFP (Enhanced Yellow Fluorescence Protein).

When the combination of fluorescent proteins is employed which consists of a cyan fluorescent protein and a yellow fluorescent protein, though each of those proteins may be fused to Epac2 either on the N-terminus or the C-terminus thereof insofar as the fusion protein thus formed causes FRET, it is preferred that a cyan fluorescent protein is fused on the N-terminus, and a yellow fluorescent protein on the C-terminus.

In the present invention, the term "the FRET technique" means the method in which a change occurring in fluorescent-labeled Epac2 is measured as a change in fluorescent light emitted by FRET. Also in the present invention, the term "fluorescent light" means the fluorescent light emitted from fluorescent-labeled Epac2 by FRET.

Though any expression vectors may be used, without any particular limitation, insofar as fluorescent-labeled Epac2 incorporated in it is expressed, expression vectors for mammalian cells are preferred. With the term "for mammalian cells", it is meant that the vector is capable of working as an expression vector in mammalian cells.

There is no particular limitation as to the cells used insofar as fluorescent-labeled Epac2 is expressed in them when they are transformed with the expression vector according to the present invention, and they may be either prokaryotic cells including E. coli, or eukaryotic cells such as yeast or mammalian cells.

When mammalian cells are used, there is no particular limitation as to the species from which the cells originate, and cells originating from human, ape, mouse or rat may be preferably used. In addition, there is no particular limitation as to what types of cells may be used, and such cells as fibroblasts, cells originating from kidney, epithelioid cells and pancreatic beta-cells may be used, and further, any of normal cells, cancerated cells, cell lines and primary culture cells may be used.

Furthermore, mammalian cells used may be either those cells which express K_{AT}p channels, such as MIN6 cells originating from pancreatic beta- cells, or those cells which do not express K_{AT}p channels, such as COS cells like COS-1 cells. However, in the case where influence of any interaction is to be avoided between SUR1 and fluorescent-labeled Epac2, cells which do not express K_{A}TP channels are preferably chosen. The term "cells which do not express K_{A}TP channels" includes not only those cells which do not express any K_{ATP} channels at all, but also such cells that express K_{ATP} channels but only in too small an amount to exert an influence on the measurements obtained using the FRET technique.

The method of screening for insulin secretion-potentiating agents may be performed by observing the changes in fluorescence using the FRET technique after, or both before and after, the cells expressing the fluorescent-labeled Epac2 are brought into contact with agents. If the fluorescent-labeled Epac2 consists of Epac2 to which a cyan fluorescent protein and a yellow fluorescent protein have been fused, the wavelength of the light that can be used as excitation light in the FRET technique is 435-445 nm, preferably 440 nm. And the fluorescent light emitted in this case has the wavelengths of 475-485 nm and 530-540 nm, in particular 480 nm and 535 nm.

Further, in the method of screening for insulin secretion-potentiating agents according to the present invention, a homogenate of cells expressing fluorescent-labeled Epac2 can also be used, as well as the fluorescent-labeled Epac2, fully or partly purified from the homogenate.

There is no particular limitation as to insulin secretion-potentiating agents which can be screened according to the present invention insofar as they potentiate insulin secretion directly or indirectly via Epac2, and they include agents for treating both type 1 and type 2 diabetes. The term of acting "directly via Epac2" means that an agent acts through direct binding to Epac2, and the term of acting "indirectly via Epac2" means that an insulin secretion-potentiating agent causes signal transmission through its action on a molecule which is associated with Epac2, like acting via proteins upstream of Epac2 in the signal transmission pathway, such as Rab3, or acting via a molecule which binds to Epac2, though the agent itself does not directly bind to Epac2.

The present invention can thus be used to perform screening for insulin secretion-potentiating agents which act via Epac2. This in reverse means that the present invention can be used to perform screening for agents which act not via Epac2 among insulin secretion-potentiating agents including known anti-diabetic drugs.

Further, the present invention can be used in screening for therapeutic agents for diabetic patients lacking K_{ATP} channels or for evaluation of their pharmacological effects. The term "diabetic patients lacking K_{ATP} channels" herein means those diabetic patients who hereditarily lack K_{ATP} channels and includes neonatal diabetic patients who have such a hereditary trait. Therapeutic agents to be screened or evaluated for pharmacological effects herein include, for example, SU agents. The present invention revealed that some SU agents exhibit their pharmacological activity via Epac2, while others not via Epac2. Those SU agents which are expected to work in treating diabetic patients lacking K_{ATP} channels are those which exhibit their pharmacological effect via Epac2, and such agents can be screened, and be evaluated for their effects, using the present invention.

### Examples

The present invention will be described in further detail with reference to examples. However, it is not intended that the present invention be limited to the examples. In particular, while the examples shown below are on mouse Epac2, the fact that mouse Epac2 and human Epac2, as mentioned, both consist of 1011 amino acids and the homology between them is not less than 98% strongly suggests their substantial equivalency in structure and function. Therefore, all the results obtained in the following examples on mouse Epac2 must be valid also for human Epac2 with substantial equivalency. Meanwhile, all the animal experiments were conducted according to the guidelines of Ethics Committee, School of Medicine, Kobe University.

Reagents:
Glibenclamide was purchased from ALEXIS (San Diego ,USA). Tolbutamide, chlorpropamide, acetohexamide, glipizide, nateglinide and
12-O-tetradecanoyl-phorbol-13-acetate (TPA) were purchased from SIGMA (Saint. Leuis, USA). Gliclazide was purchased from LKT laboratory(Saint Pole, USA). Tritium-labeled glibenclamide was purchased from PerknElmer (Waltham, USA).

Animals:
Mice lacking Epac2 were prepared by the technique disclosed in Shibasaki T.et al. Proc. Natl. Acad. Sci. USA 104, 19333-9 (2007). Wild-type mouse (C57BL/6) were employed as the control group in all the animal experiments.

Pancreatic beta-cells lacking Epac2:
Pancreatic beta-cells lacking Epac2 were obtained according to the technique disclosed in Shibasaki T. et al. Proc. Natl. Acad. Sci. USA 104, 19333-9 (2007), by isolating from the mice which were bred after crossing Epac2-lacking mice with IT6 mice, in which SV40 large T antibody is expressed under the control of human insulin gene promoter. The method for preparing IT6 mice is described in Miyazaki J, et al., Endocrinology, 127, 126-132 (1990). Further, MIN6 cells were prepared by the technique disclosed in Japanese patent application publication No. 2002-125661.

Mouse Epac2 expression vector:
Wild-type mouse Epac2 expression vector (pFLAG-Epac2: Fig. 1) and mutant-type mouse Epac2 expression vector (pFLAG-Epac2 G114E G422D: Fig. 2) employed are disclosed in Ozaki N. et.al., (2000) Nat. Cell. Biol. 2, 805-811 (Non-patent document 13). In the wild-type mouse Epac2 cDNA (SEQ ID NO:1), the first 14 bases and the last 12 bases except the stop codon (tag) are the primer sequences used in the PCR.

The vector map for pFLAT-CMV-2, which were used for construction of pFLAG-Epac2 and pFLAG-Epac2 G114E G422D, is shown in Fig. 3a, and the nucleotide sequence of its full-length DNA is shown in SEQ ID NO:5, the nucleotide sequence for whose multicloning site is shown in Fig. 3b and as SEQ ID NO:6, respectively. In the expression vectors pFLAG-Epac2 and pFLAG-Epac2 G114E G422D, Epac2 gene is inserted in the EcoRI position of pFLAT-CMV-2 vector.

Further, the cDNA sequence for the mouse mutant-type Epac2 employed above is shown as SEQ ID NO:7, and its amino acid sequence in SEQ ID NO:8, respectively.

Construction of cyan and yellow fluorescent-labeled mouse Epac2 expression vector - Step 1:
Using the above mouse Epac2 expression vector, pFLAG-Epac2, as a template, PCR was performed with primer BgIII-Epac2 (5'-agatctatggtcgctgcgca-3', SEQ ID NO:9) and primer Epac2-EcoRI (5'-gaattctggccttcgagg-3': SEQ ID NO: 10) to amplify cDNA for mouse Epac2. Using PfuDNA polymerase, the PCR procedure followed consisted of 10 cycles of (95 deg C: 30 sec, 55 deg C: 30 sec, 68 deg C: 2 min), 30 cycles of (94 deg C: 30 sec, 50 deg C: 30 sec, 68 deg C: 3 min) and then the final reaction at 68 deg C for 5 min. The mouse wild-type Epac2 cDNA (SEQ ID NO:1 as mentioned above) thus amplified was digested with BglII and EcoRI, and then inserted into an expression vector for a cyan fluorescent protein (ECFP), pECFP-C1 (Fig. 4, Clontech), which had been digested with BgIII and EcoRI, the DNA sequence for the multicloning site of which is shown in SEQ ID NO:11. This vector thus obtained was named pECFPmouse Epac2 expression vector. The cDNA sequence for the cyan fluorescent protein (ECFP) is shown in SEQ ID NO: 12, and its amino acid sequence in SEQ ID NO: 13, respectively.

Construction of cyan and yellow fluorescent-labeled mouse Epac2 expression vector - Step 2:
Using an yellow fluorescent protein expression vector, pEYFP-N1 (Fig. 5, Clontech)(the DNA sequence of the multicloning site of this vector is shown in SEQ ID NO:14), as a template, PCR was performed with primer GFP-fw (5'-atggtgagcaagggcg-3': SEQ ID NO:15) and primer GFP-rv (5'-cttgtacagctcgtccat-3':
   SEQ IDNO:16) to amplify the cDNA for the yellow fluorescent protein EYFP. Using PfuDNA polymerase, the PCR procedure followed consisted of 10 cycles of (95 deg C:30 sec, 55 deg C:30 sec, 72 deg C: 1 min), then 30 cycles of (94 deg C: 30 sec, 52 deg C: 30 sec, 68 deg C: 1 min) and then the final reaction at 68 deg C for 5 min.
   Using the cDNA for EYFP thus obtained by PCR as a template, PCR was performed with a primer EcoRI-EYFP (5'-gaattcatggtgagcaagg-3': 17) and a primer EYFP-EcoRI (5'-gaattccttgtacagctcgt-3': SEQ ID NO:18) to amplify EYFP cDNA to which was added an EcoRI site. The cDNA sequence for the yellow fluorescent protein (EYFP) is shown in SEQ ID NO: 19, and its amino acid sequence in SEQ ID NO:20, respectively.
   In the cDNA, the top 13 bases and the rearmost 14 bases except the stop codon "tga" are the primer sequences. Using PfuDNA polymerase, the PCR procedure followed consisted of 10 cycles of (95 deg C: 30 sec, 55 deg C: 30 sec, 72 deg C: 1 min), and 30 cycles of (94 deg C: 30 sec, 52 deg C: 30 sec, 68 deg C: 1 min) and then the final reaction at 68 deg C for 5 min. The EcoRI site-added EYFP cDNA thus amplified was digested with EcoRI and inserted into the aforementioned pECFP-mouse Epac2 expression vector which had been digested with EcoRI. This vector thus obtained was used as the fluorescent-labeled mouse Epac2 expression vector.

Construction of mutant-type fluorescent-labeled moue Epac2 expression vector - Step 1:
Using the aforementioned mutant-type mouse Epac2 expression vector (pFLAG-Epac2 G114E G422D) as a template, PCR was performed with primer BglII-Epac2 (5'-agatctatggtcgctgcgca-3': SEQ ID NO:9, aforementioned) and primer Epac2-EcoRI (5'-gaattctggccttcgagg-3': SEQ ID NO: 10, aforementioned) to amplify mutant -type mouse Epac2 cDNA. Using PfuDNA polymerase, the PCR procedure consisted of 10 cycles of (95 deg C: 30 sec, 55 deg C: 30 sec, 68 deg C: 2 min), and 30 cycles of (94 deg C: 30 sec, 50 deg C: 30 sec, 68 deg C: 3 min) and then the final reaction at 68 deg C for 5 min. The mutant-type mouse Epac2 cDNA thus amplified was digested with BglII and EcoRI, and then inserted into the pECFP-C1 vector (Clontech). This vector thus obtained was named the mutant-type pECFP-mouse Epac2 expression vector.

Construction of mutant-type fluorescent-labeled mouse Epac2 expression vector - Step 2:
Using the pEYFP-N1 vector (Clontech) as a template, PCR was performed with primer GFP-fw (5'-atggtgagcaagggcg-3': SEQ ID NO:15, aforementioned) and primer GFP-rv (5'-cttgtacagctcgtccat-3': SEQ ID NO: 16) to amplify the yellow fluorescent protein EYFP cDNA. Using PfuDNA polymerase, the PCR procedure followed consisted of 10 cycles of (95 deg C: 30 sec, 55 deg C: 30 sec, 72 deg C: 1 min), and 30 cycles of (94 deg C: 30 sec, 52 deg C: 30 sec, 68 deg C: 1 min) then the final reaction at 68 deg C for 5 min. Then, using the EYFP cDNA thus obtained by PCR as a template, PCR was performed with primer EcoRI-EYFP (5'-gaattcatggtgagcaagg-3': SEQ ID NO: 17, aforementioned) and primer EYFP-EcoRI (5'-gaattccttgtacagctcgt-3': SEQ ID NO:18, aforementioned) to amplify the EcoRI site-added EYFP cDNA. Using PfuDNA polymerase, the PCR procedure followed consisted of 10 cycles of (95 deg C: 30 sec, 55 deg C: 30 sec, 72 deg C: 1 min), and 30 cycles of (94 deg C: 30 sec, 52 deg C: 30 sec, 68 deg C: 1 min) and then the final reaction at 68 deg C for 5 min. The EcoRI site-added EYFP cDNA thus amplified was digested with EcoRI, and inserted into the mutant-type pECFP-mouse Epac2 expression vector which had been digested with EcoRI. The vector thus obtained was named fluorescent-labeled mutant-type mouse Epac2 expression vector.

Fig. 6 shows a schematic diagrams of the secondary structure of fluorescent-labeled mouse Epac2 and fluorescent-labeled mutant-type mouse Epac2.

Cell culture and transformation:
Culture of MIN6 and COS-1 cells were conducted in Dulbecco's modified Eagle's medium (DMEM) containing 10% heat-inactivated fetal bovine serum at 5% CO₂. Transformation of the cells was performed using FuGENE6 transfection reagent (Roche Molecular Biochemicals, Basel, Switzerland).

Observation and dynamic analysis of fluorescent-labeled mouse Epac2 by FRET technique:
Two days before the day for measurement, the cells were transformed with fluorescent-labeled mouse Epac2 expression vector and then cultured. On the day before the day for measurement, the cells were transferred onto the glass culture dishes of 25 mm in diameter, and culture was continued. About 48 hours after the transformation, the culture medium was replaced with HEPES-KRB buffer (HEPES buffer containing 133.4 mM NaCl, 4.7 mM KCl, 1.2 mM KH₂PO₄, 1.2 mM MgSO₄, 2.5 mM CaCl₂, 5.0 mM NaHCO₃, 2.8 mM glucose, and 0.2% BSA (pH 7.4)). The cells were observed through a confocal microscope (FV1000, Olympus Corporation) equipped with the UPlanSApo objective lens (100 * oil/1.40 NA) to set the focus of the lens on the cells, and excitation light with the wavelength of 440 nm was irradiated using 440 nm LD laser (FV5-LDPSU, Olympus Corporation) at the power of 0.5%. Fluorescent light emitted from thus excited fluorescent-labeled mouse Epac2 was observed through two fluorescence filters, 480DF30 (for 480 nm light) and 535DF25 (for 535 nm light) as dual images of the fluorescent light every 5 seconds for dynamic analysis of the emitted fluorescent light. The results of the dynamic analysis, after first calculating the ratio R in intensity of fluorescence at 535 nm to 480 nm at each measurement (ratio of intensity at 535 nm/intensity at 480 nm), were expressed in the value which was derived by dividing the ratio by the initial value R₀ (ratio of intensity at 535 nm/ intensity at 480 nm before addition of an agent), i.e., R/R₀. All the observation was carried out at room temperature.

Binding experiment of sulfonylureas:
COS 1 cells were transformed with mouse Epac2 expression vector. Two days after transformation, the cells were washed twice with a buffer (20 mM HEPES containing 119 mM NaCl, 4.7 mM KCl ,2.5 mM CaCl₂, 1.2 mM KH₂PO₄, 1.2 mM MgSO₄, 5.0 mM NaHCO₃, (pH 7.4)) and suspended in the same buffer at the density of 2.5-5.2 * 10⁵ cells/400 micro L. The cells were subdivided into 400 micro L each, and after addition of tritium-labeled glibenclamide, were let stand for one hour at room temperature. Unlabeled glibenclamide or tolbutamide, or the like was added at the same time and was let compete with tritium-labeled glibenclamide. The COS-1 cells were fractured, and tritium-labeled glibenclamide bound to the proteins within the cells was adsorbed onto Whatman GF/C membrane by vacuum filtration (Whatman, Maidstone, UK). The membrane was washed four times with the same buffer which had been ice cooled, and then measured for radioactivity on a liquid scintillation counter.

GTP-RAP1 pull-down assay:
GTP-RAP1 pull-down assay was performed according to the method described in Shibasaki T.et al. Proc. Natl. Acad. Sci. USA 104, 19333-9 (2007). Namely, MIN6 cells which had been let stand for 30 minutes in HEPES-KRB buffer in advance were cultured for further 15 minutes in HEPES-KRB buffer containing an agent of interest and 2.8 mM glucose. Then, the cells were fractured, and to the cell lysate thus obtained was added glutathione resin which carried GST-Ra1GDS-RID bound to it (SIGMA). After 90 minutes of incubation at 4 deg C, the resin was separated by centrifugation, washed several times with HEPES-KRB buffer, and subjected to SDS-PAGE gel electrophoresis. After the SDS-PAGE gel electrophoresis, Western blotting was carried to transfer the protein onto PVDF membrane, and Rap1 transferred on the membrane was detected with anti-Rap1 antibody (Santa Cruz Biotechnology, Inc., USA).

Insulin secretion experiment:
Mouse pancreatic beta-cells were isolated from wild-type mice (C57BL/6) or Epac2-lacking mice, and cultured for two days. The mouse pancreatic beta-cells were let stand in HEPES-KRB buffer containing 2.8 mM glucose for 30 minutes. The cells then were dispensed to 92-well plates in such a manner that five pancreatic islets of a like size were contained in each well, and cultured for 15 minutes in 100 micro L HEPES-KRB buffer containing an agent of interest and 2.8 mM glucose. The amount of insulin released in the medium and that in the cells were measured using an insulin assay kit (Medical Biological Laboratories Co. Ltd.). The amount of secreted insulin was normalized by the amount of insulin in the cells.

Oral glucose tolerance test:
Mice fasted for 16 hours were orally administered with 1.5 g/kg body weight of glucose alone or with 1.5 g/kg body weight of glucose plus 100 mg/kg body weight of tolbutamide. Peripheral blood was sampled at a predetermined point of time, and serum insulin concentration and blood glucose concentration were measured with an ELISA kit (mftd. by Morinaga) and Antsense III glucose analysis device (Bayer Yakuhin, Ltd.), respectively.

Observation of the change in three-dimensional structure of fluorescent-labeled mouse Epac2 by FRET technique:
When the COS-1 cells transformed with fluorescent-labeled mouse Epac2 expression vector (fluorescent-labeled mouse Epac2 expressing COS-1 cells) were examined using the FRET technique for 6 minutes in a buffer solution containing 1 mM or 10 mM 8-bromo-cAMP, an analogue to cAMP, a concentration-dependent decrease in R/ Ro value was observed (Fig. 7a). This decrease in R/R₀ value was thought to be the result of a change in FRET due to a change in the three-dimensional structure of the fluorescent-labeled mouse Epac2 which was caused by the binding of 8-bromo-cAMP to it. On the other hand, when the COS-1 cells transformed with the fluorescentlabeled mutant-type mouse Epac2 expression vector, whose cAMP binding site was destroyed, were examined in the same manner, no decrease in R/R₀ value was observed (Fig. 7b). This was thought to be due to 8-bromo-cAMP being unable to bind to the mutant-type fluorescent-labeled mouse Epac2, causing no change in the three-dimensional structure of the mutant-type fluorescent-labeled mouse Epac2, which led to no change in FRET. In addition, when culture was conducted without addition of 8-bromo-cAMP, no change in R/R₀ was observed in either of the cases (Fig. 7a, Fig. 7b).

These results demonstrate that the change in the higher structure of fluorescent-labeled mouse Epac2 caused by the binding of 8-bromo-cAMP to it can be observed within the cells by the FRET technique. Thus, the binding of cAMP to Epac2 is one of the steps of signal transmission via Epac2, and it can be observed over time using cells expressing fluorescent-labeled mouse Epac2.

Binding of SU agents to fluorescent-labeled mouse Epac2:
When MIN6 cells which had been transformed with the fluorescent-labeled mouse Epac2 expression vector was examined using the FRET technique in the presence of an SU agent, tolbutamide or glibenclamide, concentration-dependent decrease in R/R₀ value was observed (Fig. 8). As the SU agents are known to exhibit their function via their binding to SUR1, a component of K_{ATP} channels, a possibility couldn't be ruled out that this decrease in R/R₀ value observed here was an indirect effect through SUR1.
Thus, fluorescent-labeled mouse Epac2 expressing COS-1 cells that did not express SUR1 were examined using the FRET technique in the presence of one of various SU agents, and this revealed that a decrease in R/R₀ was observed in the presence of tolbutamide, glibenclamide, chlorpropamide, acetohexamide, and glipizide (Fig. 9a-e).
The results suggest that these SU agents act not via SUR1 but directly on Epac2 to alter the higher structure of it. This was surprising because Epac2 had never been expected to be a target of SU agents. On the other hand, no change in R/R₀ value was observed in the presence of gliclazide or nateglinide, although these are among SU agents. This suggests that these agents do not act directly on Epac2 to cause an alteration in its higher structure (Fig. 9f-g). As Epac2 is a signal transmitter which plays a role in insulin secretion, the above results suggest that there are different groups of SU agents with respect to their mechanism of action, one working via Epac2 and the other not.

Then, in order to verify whether SU agents bind directly to Epac2, a sulfonylurea binding experiment was carried out using tritium-labeled glibenclamide. The COS-1 cells transformed with mouse Epac2 expression vector were cultured in the presence of tritium-labeled glibenclamide at a concentration of 1-40 nM, and the cells then were fractured to measure the radioactivity of tritium-labeled glibenclamide bound to intracellular factors of COS-1. The amount of nonspecifically bound tritium-labeled glibenclamide was regarded as the radioactivity measured when cultured in the copresence of non-radioactive-labeled glibenclamide at the concentration of 100 micro M. As a result, the amount of specifically bound tritium-labeled glibenclamide was found increased in a concentration-dependent manner (Fig. 10a). On the other hand, no specific binding was observed with normal COS-1 cells (data not shown). These results show that glibenclamide specifically binds to Epac2.

Next, the COS-1 cells transformed with mouse Epac2 expression vector were cultured in the copresence of unlabeled glibenclamide (GLB), tolbutamide (TLB) or 8-bromo-cAMP (8-Br-cAMP) and 10 nM tritium-labeled glibenclamide to let them compete for binding to Epac2. As a result, IC₅₀ was found to be 25 nM for unlabeled glibenclamide, 240 micro M for tolbutamide, and almost no competition was observed with 8-bromo-cAMP (Fig. 10b). These results suggest that the affinity of glibenclamide for Epac2 is stronger than that of tolbutamide. In addition, they further suggest that the glibenclamide binding site in the Epac2 molecule does not overlap with the cAMP binding site.

These results indicate that it is possible to observe whether or not agents, including SU agents, bind to Epac2, using the FRET technique utilizing fluorescent-labeled Epac2 as a sensor.

Involvement of Epac2 in activation of Rap1 by SU agents:
Epac2 has the GEF activity and activates Rap1 by converting it to GTP-bound Rap 1 through its binding to GTP. This reaction constitute part of signal transmission for insulin secretion in beta-cells. Thus, it was examined whether or not Rap1 is activated with SU agents, using MIN6 cells, which originate from beta-cells. Activation of Rap 1 was measured by determining GTP-bound Rap1, using GTP-RAP1 pull-down assay. As a positive control 8-bromo-cAMP and TPA, both known as Rap1 activator compounds, were used.

As a result, tolbutamide, glibenclamide, chlorpropamide, acetohexamide, and glipizide were found to increase the amount of GTP-bound Rap 1 within the MIN6 cells in a concentration-dependent manner, thus activating Rap1 (Fig. 11a-e). Glibenclamide, however, while up to the concentration of 100 nM, increasing the amount of GTP-bound Rap1, failed to increase it at concentrations higher than that (Fig. 11b). On the other hand, gliclazide was found to have no effect on the amount of GTP-bound Rap1 within the MIN6 cells, thus activating no Rap1 (Fig. 11).

Tolbutamide, glibenclamide, chlorpropamide, acetohexamide, and glipizide, all of which activated Rap1, are SU agents with which decrease in R/R₀ value was observed using the FRET technique, whereas gliclazide, which did not activate Rap1, is an SU agent with which no decrease in R/R₀ value was observed. These results suggest that activation of Rap1 by SU agents takes place via binding of SU agents to Epac2.

Then, using Epac2-lacking pancreatic beta-cells, examination was performed as to whether Rap1 was activated by SU agents. Activation of Rap1 was measured by determining the amount of GTP-bound Rap1 using GTP-RAP1 pull-down assay. As positive controls, 8-bromo-cAMP and TPA were used, which are known to activate Rap1.

As a result, none of tolbutamide, glibenclamide, chlorpropamide, acetohexamide, glipizide, all of which had been found to activate Rap1 in MIN6 cells, activated no Rap1 in the Epac2-lacking pancreatic beta-cells (Fig. 12a). However, when similar experiments were conducted after transforming the Epac2-lacking pancreatic beta-cells with the mouse Epac2 expression vector, all of the above agents were found to activate Rap1 (Fig. 12b). These results revealed that it is via Epac2 that those SU agents activate Rap1. On the other hand, nateglinide, which had no influence on R/R₀ values as examined using the FRET technique, did not activate Rap1 even in mouse Epac2 expression vector-transformed Epac2-lacking pancreatic beta-cells (data not shown).

These results indicate that those agents observed to bind Epac2 using the FRET technique employing fluorescent-labeled Epac2 as a sensor, activate Rap1 via Epac2. Namely, the results indicate that the FRET technique, employing fluorescent-labeled Epac2 as a sensor, can be used to screen for agents which activate Rap1 via Epac2.

The effect of SU agents on insulin secretion via Epac2:
The effect of SU agents on insulin secretion via Epac2 was examined in an insulin secretion experiment using pancreatic islets. First, pancreatic islets obtained from wild-type mice and Epac2-lacking mice were stimulated with glucose, and the amount of secreted insulin was determined. As a result, there was found no significant difference in insulin secretion between the two (Fig. 13a). This was also the case when stimulation was done with KCl (Fig. 13a). Then, pancreatic islets obtained from wildtype mice and Epac2-lacking mice were stimulated with tolbutamide (100 nM) or glibenclamide (10 nM), with which lowered R/R₀ values was detected using the FRET technique, and measured the amount of secreted insulin in the same manner. As a result, after the stimulation of these agents, the pancreatic islets from Epac2-lacking mice showed markedly low insulin secretion compared with the pancreatic islets from wild-type mice (Fig. 13b, c). On the other hand, there was found no significant difference between the two groups with gliclazide (10 nM), with which no change was found in R/R₀ using the FRET technique (Fig. 13d). These results indicate that a pathway through Epac2 is necessary for those SU agents which lower R/R₀ value as determined using the FRET technique to exhibit their full pharmacological effects.

To investigate the effects of SU agents via Epac2 in vivo, the effect of tolbutamide on serum insulin and blood glucose concentrations were examined after oral administration of glucose according to oral glucose tolerance test. First, glucose alone was administered to wild-type mice and Epac2-lacking mice, and their serum insulin and blood glucose concentrations were measured, which gave no significant difference between the two groups (Fig. 14a). Next, glucose was administered together with tolbutamide, and measurement was conducted in the same manner. The result showed that the serum insulin concentration was low in the Epac2-lacking mice compared with the wild-type mice (Fig. 14b). At the same time, corresponding to the serum insulin concentration, it was shown that the blood glucose concentration was high in the Epac2-lacking mice compared with the wild-type mice (Fig. 14b).

The above results indicate that the effects of SU agents via Epac2 are exhibited also in vivo, and that activation of the Epac2/Rap1 signal transmission pathway is necessary for certain SU agents, including tolbutamide, to fully exhibit their effect. Further, it was found that because agents which activate Rap1 via Epac2 can be screened using the FRET technique employing fluorescent-labeled Epac2 as a sensor, the same method can be used to screen for agents which potentiate insulin secretion via Epac2. For example, since SU agents that can be used as agents for treating diabetic patients who lack K_{ATP} channels are limited to those which activate the Epac2/Rap1 signal transmission pathway, such agents can be screened by the method. On the contrary, agents which do not activate the Epac2/Rap1 signal transmission pathway also can be screened.

### Industrial Applicability

The present invention can be utilized as a material to perform screening for insulin secretion-potentiating agents applicable to diabetic patients, and also as a method for such screening.

### Sequence Listing Free Text

SEQ ID NO: = Mouse wild-type Epac2
SEQ ID NO: 3 = Human wild-type Epac2
SEQ ID NO: 5 = pFLAT-CMV-2 full-length DNA
SEQ ID NO: 6 = Multicloning site, pFLAT-CMV-2
SEQ ID NO: 7 = Mouse mutant Epac2 (Epac2 G114E G422D)
SEQ ID NO: 9 = Primer BgIII-Epac2
SEQ ID NO: 10 = Primer Epac2-EcoRI
SEQ ID NO: 11 = Multicloning site, pECFP-C1
SEQ ID NO: 12 = ECFP
SEQ ID NO: 13 = Synthetic Construct
SEQ ID NO: 14 = Multicloning site, pEYFP-N1
SEQ ID NO: 15 = Primer GFP-fw
SEQ ID NO: 16 = Primer GFP-rv
SEQ ID NO: 17 = Primer EcoRI-EYFP
SEQ ID NO: 18 = Primer EYFP-EcoRI
SEQ ID NO: 19 = EYFP
SEQ ID NO: 20 = Synthetic Construct

## Claims

1. A method of screening for an insulin secretion-potentiating agent comprising the steps of providing candidate compounds for an insulin secretion-potentiating agent, bringing each candidate compound into contact with a fluorescent-labeled Epac2, which is a fusion protein comprising Epac2 and two different fluorescent proteins fused thereto, wherein the two different fluorescent proteins emit fluorescent light with wavelength differing from each other, irradiating the same with the excitation light for the shorter wavelength fluorescent protein of the two different fluorescent proteins before and after the contact of the same with the candidate compound to measure the intensity of the fluorescent light emitted from each of the two different fluorescent proteins included in the fluorescent-labeled Epac2, detecting changes in the intensity ratio between the two different fluorescent lights before versus after the contact with the candidate compound, and selecting a candidate compound which caused a change as an insulin secretion-potentiating agent.

2. The method according to claim 1, wherein the two different fluorescent proteins are a cyan fluorescent protein and a yellow fluorescent protein.

3. The method according to claim 1 or 2, wherein the cyan fluorescent protein is Enhanced Cyan Fluorescence Protein and the yellow fluorescent protein is Enhanced Yellow Fluorescence Protein.

## Patentansprüche

1. Verfahren zum Screening auf ein die Insulinabsonderung förderndes Mittel, wobei das Verfahren folgende Schritte umfasst: Bereitstellen potenzieller Verbindungen für ein die Insulinabsonderung förderndes Mittel, Inkontaktbringen jeder potenziellen Verbindung mit einem fluoreszenzmarkierten Epac2, bei dem es sich um ein Fusionsprotein handelt, das Epac2 und zwei verschiedene daran fusionierte Fluoreszenzproteine enthält, wobei die zwei verschiedenen Fluoreszenzproteine fluoreszierendes Licht mit voneinander verschiedener Wellenlänge aussenden, Bestrahlen dieser mit dem Erregungslicht für das die kürzere Wellenlänge aufweisende Fluoreszenzprotein der beiden verschiedenen Fluoreszenzproteine vor und nach ihrem Kontakt mit der potenziellen Verbindung zum Messen der Intensität des fluoreszierenden Lichts, das von jedem der beiden verschiedenen Fluoreszenzproteine, die in dem fluoreszenzmarkierten Epac2 enthalten sind, ausgesendet wird, Detektieren von Veränderungen des Intensitätsverhältnisses zwischen der zwei verschiedenen fluoreszierenden Lichtern vor im Vergleich zu nach dem Kontakt mit der potenziellen Verbindung, und Auswählen einer potenziellen Verbindung, die eine Veränderung verursachte, als ein die Insulinabsonderung förderndes Mittel.

2. Verfahren nach Anspruch 1, wobei die zwei verschiedenen Fluoreszenzproteine ein Cyanfluoreszenzprotein und ein gelbes Fluoreszenzprotein sind.

3. Verfahren nach Anspruch 1 oder 2, wobei das Cyanfluoreszenzprotein ein Verstärktes Cyanfluoreszenzprotein ist und das gelbe Fluoreszenzprotein ein Verstärktes Gelbes Fluoreszenzprotein ist.

## Revendications

1. Procédé de criblage d'un agent de potentialisation de sécrétion d'insuline comprenant les étapes de la fourniture de composés candidats pour un agent de potentialisation de sécrétion d'insuline, la mise en contact de chaque composé candidat avec une Epac2 maquée par fluorescence, qui est une protéine de fusion comprenant Epac2 et deux protéines fluorescentes différentes qui y sont fusionnées, dans lequel les deux protéines fluorescentes différentes émettent de la lumière fluorescente différente l'une de l'autre, l'irradiation de celles-ci avec la lumière d'excitation pour la protéine fluorescente de longueur d'onde plus courte des deux protéines fluorescentes différentes avant et après leur contact avec le composé candidat pour mesurer l'intensité de la lumière fluorescente émise à partir de chacune des deux protéines fluorescentes différentes comprises dans la Epac2 marquée par fluorescence, la détection des changements du rapport d'intensité entre les deux lumières fluorescentes différentes avant contre après le contact avec le composé candidat, et la sélection d'un composé candidat qui a provoqué un changement comme agent de potentialisation de sécrétion d'insuline.

2. Procédé selon la revendication 1, dans lequel les deux protéines fluorescentes différentes sont une protéine fluorescente cyan et une protéine fluorescente jaune.

3. Procédé selon la revendication 1 ou 2, dans lequel la protéine fluorescente cyan est une protéine de fluorescence cyan augmentée et la protéine fluorescente jaune est une protéine de fluorescence jaune augmentée.


### SEQUENCE LISTING

<110> **KOBE UNIVERSITY JCR PHARMACEUTICALS CO., LTD.**
<120> **Method of Screening for Insulin Secretion-Potentiating Agents**
<130> GP128-PCT
<160> 20
<170> **Patent In version 3.5**
<210> 1
   <211> 3036
   <212> DNA
   <213> **Mus musculus**
<220>
   <221> CDS
   <222> (1)..(3036)
   <223> **Mouse wild-type Epac2**
<400> **1**
   atg gtc gct gcg cac gct gca cac tct cag tcc tcg gcc gag tgg atc      48
   Met Val Ala Ala His Ala Ala His Ser Gln Ser Ser Ala Glu Trp Ile
   1               5                   10                  15
   gcc tgc ctg gat aaa agg ccg ttg gag cga tct agt gaa gat gtg gac      96
   Ala Cys Leu Asp Lys Arg Pro Leu Glu Arg Ser Ser Glu Asp Val Asp
               20                  25                  30
   ata att ttc acg cgg ctg aaa gga gtt aaa gct ttt gag aaa ttt cac     144
   Ile Ile Phe Thr Arg Leu Lys Gly Val Lys Ala Phe Glu Lys Phe His
           35                  40                  45
   cca aac ctc ctt cgt cag att tgt tta tgc ggt tac tat gag aac ctg     192
   Pro Asn Leu Leu Arg Gln Ile Cys Leu Cys Gly Tyr Tyr Glu Asn Leu
       50                  55                  60
   gaa aaa gga atc aca ctg ttt cgc caa ggg gat att gga acc aac tgg     240
   Glu Lys Gly Ile Thr Leu Phe Arg Gln Gly Asp Ile Gly Thr Asn Trp
   65                  70                  75                  80
   tat gct gtc ctg gct ggg tct ttg gat gtt aaa gtg tct gag acc agc     288
   Tyr Ala Val Leu Ala Gly Ser Leu Asp Val Lys Val Ser Glu Thr Ser
                   85                  90                  95
   agt cac cag gat gcg gtg acc atc tgc act ctg gga att ggg aca gcc     336
   Ser His Gln Asp Ala Val Thr Ile Cys Thr Leu Gly Ile Gly Thr Ala
               100                 105                 110
   ttt gga gag tcc att ctg gat aac acc cct cgc cat gca acc atc gtt     384
   Phe Gly Glu Ser Ile Leu Asp Asn Thr Pro Arg His Ala Thr Ile Val
           115                 120                 125
   acc agg gag agc agc gaa ctt ctc cgc att gag cag gag gac ttc aag     432
   Thr Arg Glu Ser Ser Glu Leu Leu Arg Ile Glu Gln Glu Asp Phe Lys
       130                 135                 140
   gca cta tgg gag aaa tac cga cag tat atg gcc gga ctt ctg gct cct     480
   Ala Leu Trp Glu Lys Tyr Arg Gln Tyr Met Ala Gly Leu Leu Ala Pro
   145                 150                 155                 160
   ccc tat ggt gtt atg gaa acg ggc tct aac aat gac agg att cct gac        528
   Pro Tyr Gly Val Met Glu Thr Gly Ser Asn Asn Asp Arg Ile Pro Asp
                   165                 170                 175
   aag gag aat aca cct ctc att gaa ccc cac gtt cct ctc cgt cct gct        576
   Lys Glu Asn Thr Pro Leu Ile Glu Pro His Val Pro Leu Arg Pro Ala
               180                 185                 190
   cac acc att acc aag gtc cct tca gag aag atc ctc aga gct gga aaa        624
   His Thr Ile Thr Lys Val Pro Ser Glu Lys Ile Leu Arg Ala Gly Lys
           195                 200                 205
   att tta cga att gcc att ctc tct cga gct ccc cac atg ata aga gac        672
   Ile Leu Arg Ile Ala Ile Leu Ser Arg Ala Pro His Met Ile Arg Asp
       210                 215                 220
   aga aag tac cac cta aag aca tac aga caa tgc tgt gtt ggg act gag        720
   Arg Lys Tyr His Leu Lys Thr Tyr Arg Gln Cys Cys Val Gly Thr Glu
   225                 230                 235                 240
   ctg gta gac tgg atg ata cag cag aca tcc tgt gtt cac tcg cgg act        768
   Leu Val Asp Trp Met Ile Gln Gln Thr Ser Cys Val His Ser Arg Thr
                   245                 250                 255
   caa gct gtt ggc atg tgg caa gtc ttg ctg gaa gat ggt gtc ctc aac        816
   Gln Ala Val Gly Met Trp Gln Val Leu Leu Glu Asp Gly Val Leu Asn
               260                 265                 270
   cat gtg gac cag gag cgc cat ttc caa gac aaa tat tta ttt tat cga        864
   His Val Asp Gln Glu Arg His Phe Gln Asp Lys Tyr Leu Phe Tyr Arg
           275                 280                 285
   ttt ctg gat gac gag cgt gag gat gcc cct ttg cct act gag gaa gag        912
   Phe Leu Asp Asp Glu Arg Glu Asp Ala Pro Leu Pro Thr Glu Glu Glu
       290                 295                 300
   aag aag gag tgt gat gaa gaa ctt cag gac acc atg ctg ctg ctc tca        960
   Lys Lys Glu Cys Asp Glu Glu Leu Gln Asp Thr Met Leu Leu Leu Ser
   305                 310                 315                 320
   cag atg ggc cct gac gcc cac atg aga atg atc ctg cga aaa cca cct       1008
   Gln Met Gly Pro Asp Ala His Met Arg Met Ile Leu Arg Lys Pro Pro
                   325                 330                 335
   ggc cag agg act gtg gat gac cta gag att atc tac gac gag ctc ctt       1056
   Gly Gln Arg Thr Val Asp Asp Leu Glu Ile Ile Tyr Asp Glu Leu Leu
               340                 345                 350
   cat att aaa gcc tta tcc cat ctc tct acc aca gtg aaa cgg gag tta       1104
   His Ile Lys Ala Leu Ser His Leu Ser Thr Thr Val Lys Arg Glu Leu
           355                 360                 365
   gca ggt gtt ctc att ttt gag tct cac gcc aaa gga gga act gtg ttg       1152
   Ala Gly Val Leu Ile Phe Glu Ser His Ala Lys Gly Gly Thr Val Leu
       370                 375                 380
   ttt aac cag ggg gaa gaa ggt acc tcc tgg tac atc att ctg aaa gga       1200
   Phe Asn Gln Gly Glu Glu Gly Thr Ser Trp Tyr Ile Ile Leu Lys Gly
   385                 390                 395                 400
   tcc gtg aat gta gtc att tat ggc aag ggt gtg gtc tgc acc ctg cac       1248
   Ser Val Asn Val Val Ile Tyr Gly Lys Gly Val Val Cys Thr Leu His
                   405                 410                 415
   gaa gga gat gac ttt ggc aag tta gct cta gtg aac gat gct cca aga       1296
   Glu Gly Asp Asp Phe Gly Lys Leu Ala Leu Val Asn Asp Ala Pro Arg
               420                 425                 430
   gct gcc tcc att gtt ctt cgg gaa gat aat tgt cac ttc cta aga gtc      1344
   Ala Ala Ser Ile Val Leu Arg Glu Asp Asn Cys His Phe Leu Arg Val
           435                 440                 445
   gac aag gaa gac ttc aat cgg att ctg agg gac gtt gag gcg aat aca      1392
   Asp Lys Glu Asp Phe Asn Arg Ile Leu Arg Asp Val Glu Ala Asn Thr
       450                 455                 460
   gtc aga ctt aaa gaa cat gac caa gat gtc ttg gta ctg gag aag gtc      1440
   Val Arg Leu Lys Glu His Asp Gln Asp Val Leu Val Leu Glu Lys Val
   465                 470                 475                 480
   cca gca ggg aac aga gct gct aat caa gga aac tca cag cct cag caa      1488
   Pro Ala Gly Asn Arg Ala Ala Asn Gln Gly Asn Ser Gln Pro Gln Gln
                   485                 490                 495
   aag tat act gtg atg tca gga aca cct gaa aag att tta gag cat ttt      1536
   Lys Tyr Thr Val Met Ser Gly Thr Pro Glu Lys Ile Leu Glu His Phe
               500                 505                 510
   cta gaa aca ata cgc ctt gag cca tcg ttg aat gaa gca aca gat tcg      1584
   Leu Glu Thr Ile Arg Leu Glu Pro Ser Leu Asn Glu Ala Thr Asp Ser
           515                 520                 525
   gtt tta aat gac ttt gtt atg atg cac tgt gtt ttt atg cca aat acc      1632
   Val Leu Asn Asp Phe Val Met Met His Cys Val Phe Met Pro Asn Thr
       530                 535                 540
   cag ctt tgc cct gcc ctt gtg gcc cat tac cac gca cag cct tct caa      1680
   Gln Leu Cys Pro Ala Leu Val Ala His Tyr His Ala Gln Pro Ser Gln
   545                 550                 555                 560
   ggt acc gag cag gag aga atg gat tat gcc ctc aac aac aag agg cgg      1728
   Gly Thr Glu Gln Glu Arg Met Asp Tyr Ala Leu Asn Asn Lys Arg Arg
                   565                 570                 575
   gtc atc cgc ttg gtc ctg cag tgg gcg gcc atg tat ggc gat ctc ctc      1776
   Val Ile Arg Leu Val Leu Gln Trp Ala Ala Met Tyr Gly Asp Leu Leu
               580                 585                 590
   caa gaa gat gat gtg gcc atg gcc ttc ctg gag gag ttc tat gtg tct      1824
   Gln Glu Asp Asp Val Ala Met Ala Phe Leu Glu Glu Phe Tyr Val Ser
           595                 600                 605
   gta tca gat gac gca cgg atg atg gct gcc ttc aag gag cag ctg cca      1872
   Val Ser Asp Asp Ala Arg Met Met Ala Ala Phe Lys Glu Gln Leu Pro
       610                 615                 620
   gag ctg gag aag att gtc aag caa atc tca gaa gac gca aaa gct cca      1920
   Glu Leu Glu Lys Ile Val Lys Gln Ile Ser Glu Asp Ala Lys Ala Pro
   625                 630                 635                 640
   cag aag aag cac aag gtg ctt ttg caa cag ttc aac aca ggt gac gag      1968
   Gln Lys Lys His Lys Val Leu Leu Gln Gln Phe Asn Thr Gly Asp Glu
                   645                 650                 655
   agg gcc cag aag cgt cag cct att cgt ggc tct gat gag gtt ttg ttc      2016
   Arg Ala Gln Lys Arg Gln Pro Ile Arg Gly Ser Asp Glu Val Leu Phe
               660                 665                 670
   aag gtc tac tgc atc gac cac acc tat act acc att cgt gtg ccg gta      2064
   Lys Val Tyr Cys Ile Asp His Thr Tyr Thr Thr Ile Arg Val Pro Val
           675                 680                 685
   gct gcc tcg gtg aag gaa gtc atc agt gca gta gct gac aaa ctg ggc      2112
   Ala Ala Ser Val Lys Glu Val Ile Ser Ala Val Ala Asp Lys Leu Gly
       690                 695                 700
   tca ggg gaa ggc ctg atc atc gtc aag atg aac tct gga gga gaa aag      2160
   Ser Gly Glu Gly Leu Ile Ile Val Lys Met Asn Ser Gly Gly Glu Lys
   705                 710                 715                 720
   gtg gtg ctg aaa tct aat gat gtt tca gta ttt acg acg ctc acc att      2208
   Val Val Leu Lys Ser Asn Asp Val Ser Val Phe Thr Thr Leu Thr Ile
                   725                 730                 735
   aat gga cgc ctg ttt gcc tgc ccg aga gag caa ttc gac tca ctg act      2256
   Asn Gly Arg Leu Phe Ala Cys Pro Arg Glu Gln Phe Asp Ser Leu Thr
               740                 745                 750
   ccc ttg ccg gaa cag gaa ggc ccg acc act ggg aca gtg gga aca ttt      2304
   Pro Leu Pro Glu Gln Glu Gly Pro Thr Thr Gly Thr Val Gly Thr Phe
           755                 760                 765
   gag ctg atg agc tcg aaa gac ctg gcg tac cag atg aca acc tac gat      2352
   Glu Leu Met Ser Ser Lys Asp Leu Ala Tyr Gln Met Thr Thr Tyr Asp
       770                 775                 780
   tgg gaa ctc ttc aac tgt gtg cat gag ctg gag cta atc tac cac aca      2400
   Trp Glu Leu Phe Asn Cys Val His Glu Leu Glu Leu Ile Tyr His Thr
   785                 790                 795                 800
   ttt gga agg cat aat ttt aaa aag acc acg gca aac ttg gat ttg ttc      2448
   Phe Gly Arg His Asn Phe Lys Lys Thr Thr Ala Asn Leu Asp Leu Phe
                   805                 810                 815
   ctg agg agg ttt aat gaa att cag ttt tgg gtt gtc act gag gtc tgc      2496
   Leu Arg Arg Phe Asn Glu Ile Gln Phe Trp Val Val Thr Glu Val Cys
               820                 825                 830
   ctt tgt tcc cag ctc agc aaa cgt gtt cag ctt ttg aaa aaa ttt atc      2544
   Leu Cys Ser Gln Leu Ser Lys Arg Val Gln Leu Leu Lys Lys Phe Ile
           835                 840                 845
   aag ata gcg gct cac tgc aag gag tac aaa aat cta aat tcc ttt ttc      2592
   Lys Ile Ala Ala His Cys Lys Glu Tyr Lys Asn Leu Asn Ser Phe Phe
       850                 855                 860
   gcc atc gtc atg gga ctc agc aac gtg gcc gtg agc cgc ttg gca cta      2640
   Ala Ile Val Met Gly Leu Ser Asn Val Ala Val Ser Arg Leu Ala Leu
   865                 870                 875                 880
   acg tgg gag aaa ctg ccg agc aag ttt aag aag ttc tat gcg gag ttt      2688
   Thr Trp Glu Lys Leu Pro Ser Lys Phe Lys Lys Phe Tyr Ala Glu Phe
                   885                 890                 895
   gag agc ttg atg gat cct tcc aga aac cac agg gca tac agg ctg aca      2736
   Glu Ser Leu Met Asp Pro Ser Arg Asn His Arg Ala Tyr Arg Leu Thr
               900                 905                 910
   gca gcc aag ctg gag ccc cct ctc atc cct ttc atg ccc ttg ctt att      2784
   Ala Ala Lys Leu Glu Pro Pro Leu Ile Pro Phe Met Pro Leu Leu Ile
           915                 920                 925
   aaa gat atg aca ttt act cat gag ggg aac aag acg ttc att gac aat      2832
   Lys Asp Met Thr Phe Thr His Glu Gly Asn Lys Thr Phe Ile Asp Asn
       930                 935                 940
   cta gta aac ttt gaa aaa atg cgc atg att gca aac act gcc aga aca        2880
   Leu Val Asn Phe Glu Lys Met Arg Met Ile Ala Asn Thr Ala Arg Thr
   945                 950                 955                 960
   gta cgg tac tac agg agc cag ccc ttc aat ccg gat gcc gct caa gct        2928
   Val Arg Tyr Tyr Arg Ser Gln Pro Phe Asn Pro Asp Ala Ala Gln Ala
                   965                 970                 975
   aat aag aac cat cag gat gtc cgg agt tat gta cgg caa tta aat gtg        2976
   Asn Lys Asn His Gln Asp Val Arg Ser Tyr Val Arg Gln Leu Asn Val
               980                 985                 990
   att gac aac cag aga act tta tca  cag atg tca cac aga  tta gag cct      3024
   Ile Asp Asn Gln Arg Thr Leu Ser  Gln Met Ser His Arg  Leu Glu Pro
           995                 1000                1005
   cga agg  cca tag                                                       3036
   Arg Arg  Pro
       1010
<210> 2
   <211> 1011
   <212> PRT
   <213> Mus musculus
<400> 2
   Met Val Ala Ala His Ala Ala His Ser Gln Ser Ser Ala Glu Trp Ile
   1               5                   10                  15
   Ala Cys Leu Asp Lys Arg Pro Leu Glu Arg Ser Ser Glu Asp Val Asp
               20                  25                  30
   Ile Ile Phe Thr Arg Leu Lys Gly Val Lys Ala Phe Glu Lys Phe His
           35                  40                  45
   Pro Asn Leu Leu Arg Gln Ile Cys Leu Cys Gly Tyr Tyr Glu Asn Leu
       50                  55                  60
   Glu Lys Gly Ile Thr Leu Phe Arg Gln Gly Asp Ile Gly Thr Asn Trp
   65                  70                  75                  80
   Tyr Ala Val Leu Ala Gly Ser Leu Asp Val Lys Val Ser Glu Thr Ser
                   85                  90                  95
   Ser His Gln Asp Ala Val Thr Ile Cys Thr Leu Gly Ile Gly Thr Ala
               100                 105                 110
   Phe Gly Glu Ser Ile Leu Asp Asn Thr Pro Arg His Ala Thr Ile Val
           115                 120                 125
   Thr Arg Glu Ser Ser Glu Leu Leu Arg Ile Glu Gln Glu Asp Phe Lys
       130                 135                 140
   Ala Leu Trp Glu Lys Tyr Arg Gln Tyr Met Ala Gly Leu Leu Ala Pro
   145 150 155 160
   Pro Tyr Gly Val Met Glu Thr Gly Ser Asn Asn Asp Arg Ile Pro Asp
   165 170 175
   Lys Glu Asn Thr Pro Leu Ile Glu Pro His Val Pro Leu Arg Pro Ala
   180 185 190
   His Thr Ile Thr Lys Val Pro Ser Glu Lys Ile Leu Arg Ala Gly Lys
   195 200 205
   Ile Leu Arg Ile Ala Ile Leu Ser Arg Ala Pro His Met Ile Arg Asp
   210 215 220
   Arg Lys Tyr His Leu Lys Thr Tyr Arg Gln Cys Cys Val Gly Thr Glu
   225 230 235 240
   Leu Val Asp Trp Met Ile Gln Gln Thr Ser Cys Val His Ser Arg Thr
   245 250 255
   Gln Ala Val Gly Met Trp Gln Val Leu Leu Glu Asp Gly Val Leu Asn
   260 265 270
   His Val Asp Gln Glu Arg His Phe Gln Asp Lys Tyr Leu Phe Tyr Arg
   275 280 285
   Phe Leu Asp Asp Glu Arg Glu Asp Ala Pro Leu Pro Thr Glu Glu Glu
   290 295 300
   Lys Lys Glu Cys Asp Glu Glu Leu Gln Asp Thr Met Leu Leu Leu Ser
   305 310 315 320
   Gln Met Gly Pro Asp Ala His Met Arg Met Ile Leu Arg Lys Pro Pro
   325 330 335
   Gly Gln Arg Thr Val Asp Asp Leu Glu Ile Ile Tyr Asp Glu Leu Leu
   340 345 350
   His Ile Lys Ala Leu Ser His Leu Ser Thr Thr Val Lys Arg Glu Leu
   355 360 365
   Ala Gly Val Leu Ile Phe Glu Ser His Ala Lys Gly Gly Thr Val Leu
   370 375 380
   Phe Asn Gln Gly Glu Glu Gly Thr Ser Trp Tyr Ile Ile Leu Lys Gly
   385 390 395 400
   Ser Val Asn Val Val Ile Tyr Gly Lys Gly Val Val Cys Thr Leu His
   405 410 415
   Glu Gly Asp Asp Phe Gly Lys Leu Ala Leu Val Asn Asp Ala Pro Arg
   420 425 430
   Ala Ala Ser Ile Val Leu Arg Glu Asp Asn Cys His Phe Leu Arg Val
   435 440 445
   Asp Lys Glu Asp Phe Asn Arg Ile Leu Arg Asp Val Glu Ala Asn Thr
   450 455 460
   Val Arg Leu Lys Glu His Asp Gln Asp Val Leu Val Leu Glu Lys Val
   465 470 475 480
   Pro Ala Gly Asn Arg Ala Ala Asn Gln Gly Asn Ser Gln Pro Gln Gln
   485 490 495
   Lys Tyr Thr Val Met Ser Gly Thr Pro Glu Lys Ile Leu Glu His Phe
   500 505 510
   Leu Glu Thr Ile Arg Leu Glu Pro Ser Leu Asn Glu Ala Thr Asp Ser
   515 520 525
   Val Leu Asn Asp Phe Val Met Met His Cys Val Phe Met Pro Asn Thr
   530 535 540
   Gln Leu Cys Pro Ala Leu Val Ala His Tyr His Ala Gln Pro Ser Gln
   545 550 555 560
   Gly Thr Glu Gln Glu Arg Met Asp Tyr Ala Leu Asn Asn Lys Arg Arg
   565 570 575
   Val Ile Arg Leu Val Leu Gln Trp Ala Ala Met Tyr Gly Asp Leu Leu
   580 585 590
   Gln Glu Asp Asp Val Ala Met Ala Phe Leu Glu Glu Phe Tyr Val Ser
   595 600 605
   Val Ser Asp Asp Ala Arg Met Met Ala Ala Phe Lys Glu Gln Leu Pro
   610 615 620
   Glu Leu Glu Lys Ile Val Lys Gln Ile Ser Glu Asp Ala Lys Ala Pro
   625 630 635 640
   Gln Lys Lys His Lys Val Leu Leu Gln Gln Phe Asn Thr Gly Asp Glu
   645 650 655
   Arg Ala Gln Lys Arg Gln Pro Ile Arg Gly Ser Asp Glu Val Leu Phe
   660 665 670
   Lys Val Tyr Cys Ile Asp His Thr Tyr Thr Thr Ile Arg Val Pro Val
           675                 680                 685
   Ala Ala Ser Val Lys Glu Val Ile Ser Ala Val Ala Asp Lys Leu Gly
       690                 695                 700
   Ser Gly Glu Gly Leu Ile Ile Val Lys Met Asn Ser Gly Gly Glu Lys
   705                 710                 715                     720
   Val Val Leu Lys Ser Asn Asp Val Ser Val Phe Thr Thr Leu Thr Ile
                   725                 730                 735
   Asn Gly Arg Leu Phe Ala Cys Pro Arg Glu Gln Phe Asp Ser Leu Thr
               740                 745                 750
   Pro Leu Pro Glu Gln Glu Gly Pro Thr Thr Gly Thr Val Gly Thr Phe
           755                 760                 765
   Glu Leu Met Ser Ser Lys Asp Leu Ala Tyr Gln Met Thr Thr Tyr Asp
       770                 775                 780
   Trp Glu Leu Phe Asn Cys Val His Glu Leu Glu Leu Ile Tyr His Thr
   785                 790                 795                     800
   Phe Gly Arg His Asn Phe Lys Lys Thr Thr Ala Asn Leu Asp Leu Phe
                   805                 810                 815
   Leu Arg Arg Phe Asn Glu Ile Gln Phe Trp Val Val Thr Glu Val Cys
               820                 825                 830
   Leu Cys Ser Gln Leu Ser Lys Arg Val Gln Leu Leu Lys Lys Phe Ile
           835                 840                 845
   Lys Ile Ala Ala His Cys Lys Glu Tyr Lys Asn Leu Asn Ser Phe Phe
       850                 855                 860
   Ala Ile Val Met Gly Leu Ser Asn Val Ala Val Ser Arg Leu Ala Leu
   865                 870                 875                     880
   Thr Trp Glu Lys Leu Pro Ser Lys Phe Lys Lys Phe Tyr Ala Glu Phe
                   885                 890                 895
   Glu Ser Leu Met Asp Pro Ser Arg Asn His Arg Ala Tyr Arg Leu Thr
               900                 905                 910
   Ala Ala Lys Leu Glu Pro Pro Leu Ile Pro Phe Met Pro Leu Leu Ile
           915                 920                 925
   Lys Asp Met Thr Phe Thr His Glu Gly Asn Lys Thr Phe Ile Asp Asn
   930 935 940
   Leu Val Asn Phe Glu Lys Met Arg Met Ile Ala Asn Thr Ala Arg Thr
   945 950 955 960
   Val Arg Tyr Tyr Arg Ser Gln Pro Phe Asn Pro Asp Ala Ala Gln Ala
   965 970 975
   Asn Lys Asn His Gln Asp Val Arg Ser Tyr Val Arg Gln Leu Asn Val
   980 985 990
   Ile Asp Asn Gln Arg Thr Leu Ser Gln Met Ser His Arg Leu Glu Pro
   995 1000 1005
   Arg Arg Pro
   1010
<210> 3
   <211> 3036
   <212> DNA
   <213> Homo sapiens
<220>
   <221> CDS
   <222> (1)..(3036)
   <223> Human wild-type Epac2
<400> 3
   atg gtc gct gcg cac gct gcc cat tct tcc tcc tct gcc gag tgg atc        48
   Met Val Ala Ala His Ala Ala His Ser Ser Ser Ser Ala Glu Trp Ile
   1               5                   10                  15
   gcc tgc ctg gat aaa aga cca ctg gag cga tcc agc gaa gat gtg gat        96
   Ala Cys Leu Asp Lys Arg Pro Leu Glu Arg Ser Ser Glu Asp Val Asp
               20                  25                  30
   ata atc ttc act cga ctg aaa gaa gtt aaa gct ttt gag aaa ttt cac       144
   Ile Ile Phe Thr Arg Leu Lys Glu Val Lys Ala Phe Glu Lys Phe His
           35                  40                  45
   cca aat ctc ctt cat cag att tgc tta tgt ggt tat tat gag aat ctg       192
   Pro Asn Leu Leu His Gln Ile Cys Leu Cys Gly Tyr Tyr Glu Asn Leu
       50                  55                  60
   gaa aag gga ata aca tta ttt cgc cag ggt gat att gga aca aac tgg       240
   Glu Lys Gly Ile Thr Leu Phe Arg Gln Gly Asp Ile Gly Thr Asn Trp
   65                  70                  75                  80
   tat gct gtc ctg gca ggg tct ttg gat gtt aaa gta tct gag acc agc       288
   Tyr Ala Val Leu Ala Gly Ser Leu Asp Val Lys Val Ser Glu Thr Ser
                   85                  90                  95
   agt cac cag gat gct gtg acc atc tgt acc ctg gga att ggg acg gcc       336
   Ser His Gln Asp Ala Val Thr Ile Cys Thr Leu Gly Ile Gly Thr Ala
               100                 105                 110
   ttt gga gag tcc att ctg gac aac aca ccc cgc cat gca acc atc gtt      384
   Phe Gly Glu Ser Ile Leu Asp Asn Thr Pro Arg His Ala Thr Ile Val
           115                 120                 125
   acc agg gag agc agt gaa ttg ctc cgc atc gag cag aag gac ttc aag      432
   Thr Arg Glu Ser Ser Glu Leu Leu Arg Ile Glu Gln Lys Asp Phe Lys
       130                 135                 140
   gca cta tgg gag aaa tat cga cag tat atg gca gga ctt ctg gct cct      480
   Ala Leu Trp Glu Lys Tyr Arg Gln Tyr Met Ala Gly Leu Leu Ala Pro
   145                 150                 155                 160
   cct tat ggt gtt atg gaa acg ggc tct aac aat gac agg att cct gac      528
   Pro Tyr Gly Val Met Glu Thr Gly Ser Asn Asn Asp Arg Ile Pro Asp
                   165                 170                 175
   aag gag aac aca cct ctc att gaa cct cac gtt cct ctt cgt cct gct      576
   Lys Glu Asn Thr Pro Leu Ile Glu Pro His Val Pro Leu Arg Pro Ala
               180                 185                 190
   aac acc att acc aag gtc cct tca gag aag atc ctc aga gct gga aaa      624
   Asn Thr Ile Thr Lys Val Pro Ser Glu Lys Ile Leu Arg Ala Gly Lys
           195                 200                 205
   att tta cga aat gcc att ctc tct cga gca cct cac atg ata aga gat      672
   Ile Leu Arg Asn Ala Ile Leu Ser Arg Ala Pro His Met Ile Arg Asp
       210                 215                 220
   aga aaa tac cac cta aag aca tac aga caa tgc tgt gtg gga act gaa      720
   Arg Lys Tyr His Leu Lys Thr Tyr Arg Gln Cys Cys Val Gly Thr Glu
   225                 230                 235                 240
   ctg gtg gac tgg atg atg cag cag aca cca tgt gtt cac tcc cgg act      768
   Leu Val Asp Trp Met Met Gln Gln Thr Pro Cys Val His Ser Arg Thr
                   245                 250                 255
   caa gct gtt ggc atg tgg caa gtc ctg tta gaa gat ggt gtt ctc aac      816
   Gln Ala Val Gly Met Trp Gln Val Leu Leu Glu Asp Gly Val Leu Asn
               260                 265                 270
   cac gtg gac cag gag cac cat ttc caa gac aaa tat tta ttc tat cga      864
   His Val Asp Gln Glu His His Phe Gln Asp Lys Tyr Leu Phe Tyr Arg
           275                 280                 285
   ttt ctg gat gat gag cac gag gat gcc cct ttg cct act gag gag gag      912
   Phe Leu Asp Asp Glu His Glu Asp Ala Pro Leu Pro Thr Glu Glu Glu
       290                 295                 300
   aag aag gag tgt gat gag gag ctc cag gac acc atg ctg ctg ctg tca      960
   Lys Lys Glu Cys Asp Glu Glu Leu Gln Asp Thr Met Leu Leu Leu Ser
   305                 310                 315                 320
   cag atg ggc ccc gac gcc cac atg agg atg atc ctt cgc aaa cca cct     1008
   Gln Met Gly Pro Asp Ala His Met Arg Met Ile Leu Arg Lys Pro Pro
                   325                 330                 335
   ggc cag agg act gtg gat gac cta gag att atc tat gag gag ctt ctt     1056
   Gly Gln Arg Thr Val Asp Asp Leu Glu Ile Ile Tyr Glu Glu Leu Leu
               340                 345                 350
   cat att aaa gcc tta tcc cat ctt tct acc aca gtg aaa cga gag tta     1104
   His Ile Lys Ala Leu Ser His Leu Ser Thr Thr Val Lys Arg Glu Leu
           355                 360                 365
   gca ggt gtt ctc att ttt gag tct cac gcc aaa gga ggg act gtg ttg     1152
   Ala Gly Val Leu Ile Phe Glu Ser His Ala Lys Gly Gly Thr Val Leu
       370                 375                 380
   ttt aac cag ggg gaa gaa ggt acc tcc tgg tac att att cta aaa gga      1200
   Phe Asn Gln Gly Glu Glu Gly Thr Ser Trp Tyr Ile Ile Leu Lys Gly
   385                 390                 395                 400
   tca gtg aat gta gtc att tac ggc aag ggt gtg gtc tgc acc ctg cat      1248
   Ser Val Asn Val Val Ile Tyr Gly Lys Gly Val Val Cys Thr Leu His
                   405                 410                 415
   gaa gga gat gac ttc ggc aag tta gca cta gtg aat gat gcc cca cga      1296
   Glu Gly Asp Asp Phe Gly Lys Leu Ala Leu Val Asn Asp Ala Pro Arg
               420                 425                 430
   gct gcc tct atc gtc tta cga gaa gat aac tgc cat ttc tta aga gta      1344
   Ala Ala Ser Ile Val Leu Arg Glu Asp Asn Cys His Phe Leu Arg Val
           435                 440                 445
   gac aag gag gat ttc aac cgg atc cta agg gac gtg gag gcg aat aca      1392
   Asp Lys Glu Asp Phe Asn Arg Ile Leu Arg Asp Val Glu Ala Asn Thr
       450                 455                 460
   gtc aga ctt aaa gaa cat gac caa gat gtc ttg gtg ctg gag aag gtc      1440
   Val Arg Leu Lys Glu His Asp Gln Asp Val Leu Val Leu Glu Lys Val
   465                 470                 475                 480
   cca gca ggg aac aga gct tct aat caa gga aac tca cag cct cag caa      1488
   Pro Ala Gly Asn Arg Ala Ser Asn Gln Gly Asn Ser Gln Pro Gln Gln
                   485                 490                 495
   aag tat act gtg atg tca gga aca cct gaa aaa att tta gag cat ttt      1536
   Lys Tyr Thr Val Met Ser Gly Thr Pro Glu Lys Ile Leu Glu His Phe
               500                 505                 510
   cta gaa aca ata cgc ctt gag gca act tta aat gaa gca aca gat tct      1584
   Leu Glu Thr Ile Arg Leu Glu Ala Thr Leu Asn Glu Ala Thr Asp Ser
           515                 520                 525
   gtt tta aat gac ttt att atg atg cac tgt gtt ttt atg cca aat acc      1632
   Val Leu Asn Asp Phe Ile Met Met His Cys Val Phe Met Pro Asn Thr
       530                 535                 540
   cag ctt tgc ccg gca ctg gtg gcc cac tac cac gca cag cct tca caa      1680
   Gln Leu Cys Pro Ala Leu Val Ala His Tyr His Ala Gln Pro Ser Gln
   545                 550                 555                 560
   ggt aca gaa cag gag aaa atg gat tat gcc ctc aac aat aag agg cga      1728
   Gly Thr Glu Gln Glu Lys Met Asp Tyr Ala Leu Asn Asn Lys Arg Arg
                   565                 570                 575
   gtc atc cgc ctg gtt cta cag tgg gct gcc atg tat gga gac ctc ctg      1776
   Val Ile Arg Leu Val Leu Gln Trp Ala Ala Met Tyr Gly Asp Leu Leu
               580                 585                 590
   caa gag gat gac gtg tct atg gcc ttc ctg gag gag ttt tat gta tct      1824
   Gln Glu Asp Asp Val Ser Met Ala Phe Leu Glu Glu Phe Tyr Val Ser
           595                 600                 605
   gta tca gat gat gcc cgg atg att gct gcc ctc aag gag caa ctg cca      1872
   Val Ser Asp Asp Ala Arg Met Ile Ala Ala Leu Lys Glu Gln Leu Pro
       610                 615                 620
   gag ttg gag aag att gtc aag caa atc tca gaa gat gca aag gca cca      1920
   Glu Leu Glu Lys Ile Val Lys Gln Ile Ser Glu Asp Ala Lys Ala Pro
   625                 630                 635                 640
   caa aag aag cac aag gtt ctt ttg caa cag ttc aat acg ggc gat gag      1968
   Gln Lys Lys His Lys Val Leu Leu Gln Gln Phe Asn Thr Gly Asp Glu
                   645                 650                 655
   aga gcc cag aag cgc cag cct atc cgc ggc tct gat gaa gtt ctg ttt      2016
   Arg Ala Gln Lys Arg Gln Pro Ile Arg Gly Ser Asp Glu Val Leu Phe
               660                 665                 670
   aag gtc tat tgc atg gac cac acc tac aca acc att cgg gtg cca gtg      2064
   Lys Val Tyr Cys Met Asp His Thr Tyr Thr Thr Ile Arg Val Pro Val
           675                 680                 685
   gcc act tcg gtg aag gaa gtc atc agt gca gtt gcc gac aag ctg ggc      2112
   Ala Thr Ser Val Lys Glu Val Ile Ser Ala Val Ala Asp Lys Leu Gly
       690                 695                 700
   tcc ggg gag ggc ctg atc ata gtc aag atg agt tcc gga gga gaa aag      2160
   Ser Gly Glu Gly Leu Ile Ile Val Lys Met Ser Ser Gly Gly Glu Lys
   705                 710                 715                 720
   gtg gtg ctc aaa cct aat gat gtt tca gta ttt acg acg ctc acc att      2208
   Val Val Leu Lys Pro Asn Asp Val Ser Val Phe Thr Thr Leu Thr Ile
                   725                 730                 735
   aat gga cgc ctg ttt gct tgc ccg cga gag caa ttc gat tca ctg act      2256
   Asn Gly Arg Leu Phe Ala Cys Pro Arg Glu Gln Phe Asp Ser Leu Thr
               740                 745                 750
   ccc tta cca gaa cag gaa ggc cca act gtt gga aca gtg gga act ttt      2304
   Pro Leu Pro Glu Gln Glu Gly Pro Thr Val Gly Thr Val Gly Thr Phe
           755                 760                 765
   gaa ctg atg agc tcc aaa gat tta gca tac cag atg aca att tat gat      2352
   Glu Leu Met Ser Ser Lys Asp Leu Ala Tyr Gln Met Thr Ile Tyr Asp
       770                 775                 780
   tgg gaa ctc ttc aac tgc gtg cat gag ctg gag cta atc tat cac aca      2400
   Trp Glu Leu Phe Asn Cys Val His Glu Leu Glu Leu Ile Tyr His Thr
   785                 790                 795                 800
   ttt gga agg cat aat ttt aaa aag acc aca gca aac ttg gat ttg ttc      2448
   Phe Gly Arg His Asn Phe Lys Lys Thr Thr Ala Asn Leu Asp Leu Phe
                   805                 810                 815
   ctg agg aga ttt aat gaa att cag ttt tgg gtc gtc act gag atc tgc      2496
   Leu Arg Arg Phe Asn Glu Ile Gln Phe Trp Val Val Thr Glu Ile Cys
               820                 825                 830
   ctt tgt tct cag ctc agc aag cgt gtt cag cta tta aaa aaa ttt att      2544
   Leu Cys Ser Gln Leu Ser Lys Arg Val Gln Leu Leu Lys Lys Phe Ile
           835                 840                 845
   aag ata gca gcc cac tgt aag gag tat aaa aat ctg aat tcc ttt ttt      2592
   Lys Ile Ala Ala His Cys Lys Glu Tyr Lys Asn Leu Asn Ser Phe Phe
       850                 855                 860
   gcc atc gtc atg gga cta agt aac gtt gct gtg agc cgc ttg gca cta      2640
   Ala Ile Val Met Gly Leu Ser Asn Val Ala Val Ser Arg Leu Ala Leu
   865                 870                 875                 880
   acg tgg gag aaa ctg cca agc aag ttc aag aag ttc tat gcg gag ttt      2688
   Thr Trp Glu Lys Leu Pro Ser Lys Phe Lys Lys Phe Tyr Ala Glu Phe
                   885                 890                 895
   gaa agt tta atg gac cct tca agg aac cac agg gcc tac agg ctg aca        2736
   Glu Ser Leu Met Asp Pro Ser Arg Asn His Arg Ala Tyr Arg Leu Thr
               900                 905                 910
   gta gct aag ctg gaa cct cct ctc atc ccc ttc atg cct ttg ctc att        2784
   Val Ala Lys Leu Glu Pro Pro Leu Ile Pro Phe Met Pro Leu Leu Ile
           915                 920                 925
   aaa gat atg aca ttt act cat gag ggg aac aag acg ttc att gac aat        2832
   Lys Asp Met Thr Phe Thr His Glu Gly Asn Lys Thr Phe Ile Asp Asn
       930                 935                 940
   cta gta aac ttt gaa aaa atg cgc atg att gca aat acg gcc aga aca        2880
   Leu Val Asn Phe Glu Lys Met Arg Met Ile Ala Asn Thr Ala Arg Thr
   945                 950                 955                 960
   gtg aga tac tac agg agc caa ccc ttc aat cct gat gca gct caa gct        2928
   Val Arg Tyr Tyr Arg Ser Gln Pro Phe Asn Pro Asp Ala Ala Gln Ala
                   965                 970                 975
   aat aag aac cat cag gat gtc cgg agt tat gta cgg caa tta aat gtg        2976
   Asn Lys Asn His Gln Asp Val Arg Ser Tyr Val Arg Gln Leu Asn Val
               980                 985                 990
   att gac aac cag aga act tta tca  cag atg tca cac aga  tta gag cct      3024
   Ile Asp Asn Gln Arg Thr Leu Ser  Gln Met Ser His Arg  Leu Glu Pro
           995                 1000                1005
   cgt cga  cca tag                                                       3036
   Arg Arg  Pro
       1010
<210> 4
   <211> 1011
   <212> PRT
   <213> Homo sapiens
<400> 4
   Met Val Ala Ala His Ala Ala His Ser Ser Ser Ser Ala Glu Trp Ile
   1               5                   10                  15
   Ala Cys Leu Asp Lys Arg Pro Leu Glu Arg Ser Ser Glu Asp Val Asp
               20                  25                  30
   Ile Ile Phe Thr Arg Leu Lys Glu Val Lys Ala Phe Glu Lys Phe His
           35                  40                  45
   Pro Asn Leu Leu His Gln Ile Cys Leu Cys Gly Tyr Tyr Glu Asn Leu
       50                  55                  60
   Glu Lys Gly Ile Thr Leu Phe Arg Gln Gly Asp Ile Gly Thr Asn Trp
   65                  70                  75                  80
   Tyr Ala Val Leu Ala Gly Ser Leu Asp Val Lys Val Ser Glu Thr Ser
                   85                  90                  95
   Ser His Gln Asp Ala Val Thr Ile Cys Thr Leu Gly Ile Gly Thr Ala
               100                 105                 110
   Phe Gly Glu Ser Ile Leu Asp Asn Thr Pro Arg His Ala Thr Ile Val
           115                 120                 125
   Thr Arg Glu Ser Ser Glu Leu Leu Arg Ile Glu Gln Lys Asp Phe Lys
       130                 135                 140
   Ala Leu Trp Glu Lys Tyr Arg Gln Tyr Met Ala Gly Leu Leu Ala Pro
   145                 150                 155                 160
   Pro Tyr Gly Val Met Glu Thr Gly Ser Asn Asn Asp Arg Ile Pro Asp
                   165                 170                 175
   Lys Glu Asn Thr Pro Leu Ile Glu Pro His Val Pro Leu Arg Pro Ala
               180                 185                 190
   Asn Thr Ile Thr Lys Val Pro Ser Glu Lys Ile Leu Arg Ala Gly Lys
           195                 200                 205
   Ile Leu Arg Asn Ala Ile Leu Ser Arg Ala Pro His Met Ile Arg Asp
       210                 215                 220
   Arg Lys Tyr His Leu Lys Thr Tyr Arg Gln Cys Cys Val Gly Thr Glu
   225                 230                 235                 240
   Leu Val Asp Trp Met Met Gln Gln Thr Pro Cys Val His Ser Arg Thr
                   245                 250                 255
   Gln Ala Val Gly Met Trp Gln Val Leu Leu Glu Asp Gly Val Leu Asn
               260                 265                 270
   His Val Asp Gln Glu His His Phe Gln Asp Lys Tyr Leu Phe Tyr Arg
           275                 280                 285
   Phe Leu Asp Asp Glu His Glu Asp Ala Pro Leu Pro Thr Glu Glu Glu
       290                 295                 300
   Lys Lys Glu Cys Asp Glu Glu Leu Gln Asp Thr Met Leu Leu Leu Ser
   305                 310                 315                 320
   Gln Met Gly Pro Asp Ala His Met Arg Met Ile Leu Arg Lys Pro Pro
                   325                 330                 335
   Gly Gln Arg Thr Val Asp Asp Leu Glu Ile Ile Tyr Glu Glu Leu Leu
               340                 345                 350
   His Ile Lys Ala Leu Ser His Leu Ser Thr Thr Val Lys Arg Glu Leu
   355 360 365
   Ala Gly Val Leu Ile Phe Glu Ser His Ala Lys Gly Gly Thr Val Leu
   370 375 380
   Phe Asn Gln Gly Glu Glu Gly Thr Ser Trp Tyr Ile Ile Leu Lys Gly
   385 390 395 400
   Ser Val Asn Val Val Ile Tyr Gly Lys Gly Val Val Cys Thr Leu His
   405 410 415
   Glu Gly Asp Asp Phe Gly Lys Leu Ala Leu Val Asn Asp Ala Pro Arg
   420 425 430
   Ala Ala Ser Ile Val Leu Arg Glu Asp Asn Cys His Phe Leu Arg Val
   435 440 445
   Asp Lys Glu Asp Phe Asn Arg Ile Leu Arg Asp Val Glu Ala Asn Thr
   450 455 460
   Val Arg Leu Lys Glu His Asp Gln Asp Val Leu Val Leu Glu Lys Val
   465 470 475 480
   Pro Ala Gly Asn Arg Ala Ser Asn Gln Gly Asn Ser Gln Pro Gln Gln
   485 490 495
   Lys Tyr Thr Val Met Ser Gly Thr Pro Glu Lys Ile Leu Glu His Phe
   500 505 510
   Leu Glu Thr Ile Arg Leu Glu Ala Thr Leu Asn Glu Ala Thr Asp Ser
   515 520 525
   Val Leu Asn Asp Phe Ile Met Met His Cys Val Phe Met Pro Asn Thr
   530 535 540
   Gln Leu Cys Pro Ala Leu Val Ala His Tyr His Ala Gln Pro Ser Gln
   545 550 555 560
   Gly Thr Glu Gln Glu Lys Met Asp Tyr Ala Leu Asn Asn Lys Arg Arg
   565 570 575
   Val Ile Arg Leu Val Leu Gln Trp Ala Ala Met Tyr Gly Asp Leu Leu
   580 585 590
   Gln Glu Asp Asp Val Ser Met Ala Phe Leu Glu Glu Phe Tyr Val Ser
   595 600 605
   Val Ser Asp Asp Ala Arg Met Ile Ala Ala Leu Lys Glu Gln Leu Pro
   610 615 620
   Glu Leu Glu Lys Ile Val Lys Gln Ile Ser Glu Asp Ala Lys Ala Pro
   625 630 635 640
   Gln Lys Lys His Lys Val Leu Leu Gln Gln Phe Asn Thr Gly Asp Glu
   645 650 655
   Arg Ala Gln Lys Arg Gln Pro Ile Arg Gly Ser Asp Glu Val Leu Phe
   660 665 670
   Lys Val Tyr Cys Met Asp His Thr Tyr Thr Thr Ile Arg Val Pro Val
   675 680 685
   Ala Thr Ser Val Lys Glu Val Ile Ser Ala Val Ala Asp Lys Leu Gly
   690 695 700
   Ser Gly Glu Gly Leu Ile Ile Val Lys Met Ser Ser Gly Gly Glu Lys
   705 710 715 720
   Val Val Leu Lys Pro Asn Asp Val Ser Val Phe Thr Thr Leu Thr Ile
   725 730 735
   Asn Gly Arg Leu Phe Ala Cys Pro Arg Glu Gln Phe Asp Ser Leu Thr
   740 745 750
   Pro Leu Pro Glu Gln Glu Gly Pro Thr Val Gly Thr Val Gly Thr Phe
   755 760 765
   Glu Leu Met Ser Ser Lys Asp Leu Ala Tyr Gln Met Thr Ile Tyr Asp
   770 775 780
   Trp Glu Leu Phe Asn Cys Val His Glu Leu Glu Leu Ile Tyr His Thr
   785 790 795 800
   Phe Gly Arg His Asn Phe Lys Lys Thr Thr Ala Asn Leu Asp Leu Phe
   805 810 815
   Leu Arg Arg Phe Asn Glu Ile Gln Phe Trp Val Val Thr Glu Ile Cys
   820 825 830
   Leu Cys Ser Gln Leu Ser Lys Arg Val Gln Leu Leu Lys Lys Phe Ile
   835 840 845
   Lys Ile Ala Ala His Cys Lys Glu Tyr Lys Asn Leu Asn Ser Phe Phe
   850 855 860
   Ala Ile Val Met Gly Leu Ser Asn Val Ala Val Ser Arg Leu Ala Leu
   865 870 875 880
   Thr Trp Glu Lys Leu Pro Ser Lys Phe Lys Lys Phe Tyr Ala Glu Phe
                   885                 890                 895
   Glu Ser Leu Met Asp Pro Ser Arg Asn His Arg Ala Tyr Arg Leu Thr
               900                 905                 910
   Val Ala Lys Leu Glu Pro Pro Leu Ile Pro Phe Met Pro Leu Leu Ile
           915                 920                 925
   Lys Asp Met Thr Phe Thr His Glu Gly Asn Lys Thr Phe Ile Asp Asn
       930                 935                 940
   Leu Val Asn Phe Glu Lys Met Arg Met Ile Ala Asn Thr Ala Arg Thr
   945                 950                 955                 960
   Val Arg Tyr Tyr Arg Ser Gln Pro Phe Asn Pro Asp Ala Ala Gln Ala
                   965                 970                 975
   Asn Lys Asn His Gln Asp Val Arg Ser Tyr Val Arg Gln Leu Asn Val
               980                 985                 990
   Ile Asp Asn Gln Arg Thr Leu Ser  Gln Met Ser His Arg  Leu Glu Pro
           995                 1000                1005
   Arg Arg  Pro
       1010
<210> 5
   <211> 4679
   <212> DNA
   <213> Artificial
<220>
   <223> pFLAT-CMV-2 full-length DNA
<400> 5
   ccattcgcca ttcaggctgc gcaactgttg ggaagggcga tcggtgcggg cctcttcgct      60
   attacgccag ctggcgaaag ggggatgtgc tgcaaggcga ttaagttggg taacgccagg     120
   gttttcccag tcacgacgtt gtaaaacgac ggccagtgcc aagctgatct atacattgaa     180
   tcaatattgg caattagcca tattagtcat tggttatata gcataaatca atattggcta     240
   ttggccattg catacgttgt atctatatca taatatgtac atttatattg gctcatgtcc     300
   aatatgaccg ccatgttgac attgattatt gactagttat taatagtaat caattacggg     360
   gtcattagtt catagcccat atatggagtt ccgcgttaca taacttacgg taaatggccc     420
   gcctggctga ccgcccaacg acccccgccc attgacgtca ataatgacgt atgttcccat     480
   agtaacgcca atagggactt tccattgacg tcaatgggtg gagtatttac ggtaaactgc     540
   ccacttggca gtacatcaag tgtatcatat gccaagtccg ccccctattg acgtcaatga     600
   cggtaaatgg cccgcctggc attatgccca gtacatgacc ttacgggact ttcctacttg 660
   gcagtacatc tacgtattag tcatcgctat taccatggtg atgcggtttt ggcagtacac 720
   caatgggcgt ggatagcggt ttgactcacg gggatttcca agtctccacc ccattgacgt 780
   caatgggagt ttgttttggc accaaaatca acgggacttt ccaaaatgtc gtaataaccc 840
   cgccccgttg acgcaaatgg gcggtaggcg tgtacggtgg gaggtctata taagcagagc 900
   tcgtttagtg aaccgtcaga attgatctac catggactac aaagacgatg acgacaagct 960
   tgcggccgcg aattcatcga tagatctgat atcggtacca gtcgactcta gaggatcccg 1020
   ggtggcatcc ctgtgacccc tccccagtgc ctctcctggc cctggaagtt gccactccag 1080
   tgcccaccag ccttgtccta ataaaattaa gttgcatcat tttgtctgac taggtgtcct 1140
   tctataatat tatggggtgg aggggggtgg tatggagcaa ggggcaagtt gggaagacaa 1200
   cctgtagggc ctgcggggtc tattgggaac caagctggag tgcagtggca caatcttggc 1260
   tcactgcaat ctccgcctcc tgggttcaag cgattctcct gcctcagcct cccgagttgt 1320
   tgggattcca ggcatgcatg accaggctca gctaattttt gtttttttgg tagagacggg 1380
   gtttcaccat attggccagg ctggtctcca actcctaatc tcaggtgatc tacccacctt 1440
   ggcctcccaa attgctggga ttacaggcgt gaaccactgc tcccttccct gtccttctga 1500
   ttttaaaata actataccag caggaggacg tccagacaca gcataggcta cctggccatg 1560
   cccaaccggt gggacatttg agttgcttgc ttggcactgt cctctcatgc gttgggtcca 1620
   ctcagtagat gcctgttgaa ttgggtacgc ggccagcttg gctgtggaat gtgtgtcagt 1680
   tagggtgtgg aaagtcccca ggctccccag caggcagaag tatgcaaagc atgcatctca 1740
   attagtcagc aaccaggtgt ggaaagtccc caggctcccc agcaggcaga agtatgcaaa 1800
   gcatgcatct caattagtca gcaaccatag tcccgcccct aactccgccc atcccgcccc 1860
   taactccgcc cagttccgcc cattctccgc cccatggctg actaattttt tttatttatg 1920
   cagaggccga ggccgcctcg gcctctgagc tattccagaa gtagtgagga ggcttttttg 1980
   gaggcctagg cttttgcaaa aagctcctcg aggaactgaa aaaccagaaa gttaattccc 2040
   tatagtgagt cgtattaaat tcgtaatcat gtcatagctg tttcctgtgt gaaattgtta 2100
   tccgctcaca attccacaca acatacgagc cggaagcata aagtgtaaag cctggggtgc 2160
   ctaatgagtg agctaactca cattaattgc gttgcgctca ctgcccgctt tccagtcggg 2220
   aaacctgtcg tgccagctgc attaatgaat cggccaacgc gcggggagag gcggtttgcg 2280
   tattgggcgc tcttccgctt cctcgctcac tgactcgctg cgctcggtcg ttcggctgcg 2340
   gcgagcggta tcagctcact caaaggcggt aatacggtta tccacagaat caggggataa 2400
   cgcaggaaag aacatgtgag caaaaggcca gcaaaaggcc aggaaccgta aaaaggccgc 2460
   gttgctggcg tttttccata ggctccgccc ccctgacgag catcacaaaa atcgacgctc 2520
   aagtcagagg tggcgaaacc cgacaggact ataaagatac caggcgtttc cccctggaag 2580
   ctccctcgtg cgctctcctg ttccgaccct gccgcttacc ggatacctgt ccgcctttct 2640
   cccttcggga agcgtggcgc tttctcatag ctcacgctgt aggtatctca gttcggtgta 2700
   ggtcgttcgc tccaagctgg gctgtgtgca cgaacccccc gttcagcccg accgctgcgc 2760
   cttatccggt aactatcgtc ttgagtccaa cccggtaaga cacgacttat cgccactggc 2820
   agcagccact ggtaacagga ttagcagagc gaggtatgta ggcggtgcta cagagttctt 2880
   gaagtggtgg cctaactacg gctacactag aagaacagta tttggtatct gcgctctgct 2940
   gaagccagtt accttcggaa aaagagttgg tagctcttga tccggcaaac aaaccaccgc 3000
   tggtagcggt ggtttttttg tttgcaagca gcagattacg cgcagaaaaa aaggatctca 3060
   agaagatcct ttgatctttt ctacggggtc tgacgctcag tggaacgaaa actcacgtta 3120
   agggattttg gtcatgagat tatcaaaaag gatcttcacc tagatccttt taaattaaaa 3180
   atgaagtttt aaatcaatct aaagtatata tgagtaaact tggtctgaca gttaccaatg 3240
   cttaatcagt gaggcaccta tctcagcgat ctgtctattt cgttcatcca tagttgcctg 3300
   actccccgtc gtgtagataa ctacgatacg ggagggctta ccatctggcc ccagtgctgc 3360
   aatgataccg cgagacccac gctcaccggc tccagattta tcagcaataa accagccagc 3420
   cggaagggcc gagcgcagaa gtggtcctgc aactttatcc gcctccatcc agtctattaa 3480
   ttgttgccgg gaagctagag taagtagttc gccagttaat agtttgcgca acgttgttgc 3540
   cattgctaca ggcatcgtgg tgtcacgctc gtcgtttggt atggcttcat tcagctccgg 3600
   ttcccaacga tcaaggcgag ttacatgatc ccccatgttg tgcaaaaaag cggttagctc 3660
   cttcggtcct ccgatcgttg tcagaagtaa gttggccgca gtgttatcac tcatggttat 3720
   ggcagcactg cataattctc ttactgtcat gccatccgta agatgctttt ctgtgactgg 3780
   tgagtactca accaagtcat tctgagaata gtgtatgcgg cgaccgagtt gctcttgccc 3840
   ggcgtcaata cgggataata ccgcgccaca tagcagaact ttaaaagtgc tcatcattgg 3900
   aaaacgttct tcggggcgaa aactctcaag gatcttaccg ctgttgagat ccagttcgat 3960
   gtaacccact cgtgcaccca actgatcttc agcatctttt actttcacca gcgtttctgg 4020
   gtgagcaaaa acaggaaggc aaaatgccgc aaaaaaggga ataagggcga cacggaaatg 4080
   ttgaatactc atactcttcc tttttcaata ttattgaagc atttatcagg gttattgtct 4140
   catgagcgga tacatatttg aatgtattta gaaaaataaa caaatagggg ttccgcgcac 4200
   atttccccga aaagtgccac ctgacgcgcc ctgtagcggc gcattaagcg cggcgggtgt 4260
   ggtggttacg cgcagcgtga ccgctacact tgccagcgcc ctagcgcccg ctcctttcgc 4320
   tttcttccct tcctttctcg ccacgttcgc cggctttccc cgtcaagctc taaatcgggg 4380
   gctcccttta gggttccgat ttagtgcttt acggcacctc gaccccaaaa aacttgatta 4440
   gggtgatggt tcacgtagtg ggccatcgcc ctgatagacg gtttttcgcc ctttgacgtt 4500
   ggagtccacg ttctttaata gtggactctt gttccaaact ggaacaacac tcaaccctat 4560
   ctcggtctat tcttttgatt tataagggat tttgccgatt tcggcctatt ggttaaaaaa 4620
   tgagctgatt taacaaaaat ttaacgcgaa ttttaacaaa atattaacgc ttacaattt 4679
<210> 6
   <211> 91
   <212> DNA
   <213> Artificial
<220>
   <223> Multicloning site, pFLAT-CMV-2
<400> 6
   atggactaca aagacgatga cgacaagctt gcggccgcga attcatcgat agatctgata 60
   tcggtaccag tcgactctag aggatcccgg g 91
<210> 7
   <211> 3036
   <212> DNA
   <213> **Mus musculus**
<220>
   <221> CDS
   <222> (1)..(3036)
   <223> **Mouse mutant Epac2 (Epac2 G114E G422D)**
<400> 7
   atg gtc gct gcg cac gct gca cac tct cag tcc tcg gcc gag tgg atc      48
   Met Val Ala Ala His Ala Ala His Ser Gln Ser Ser Ala Glu Trp Ile
   1               5                   10                  15
   gcc tgc ctg gat aaa agg ccg ttg gag cga tct agt gaa gat gtg gac      96
   Ala Cys Leu Asp Lys Arg Pro Leu Glu Arg Ser Ser Glu Asp Val Asp
               20                  25                  30
   ata att ttc acg cgg ctg aaa gga gtt aaa gct ttt gag aaa ttt cac      144
   Ile Ile Phe Thr Arg Leu Lys Gly Val Lys Ala Phe Glu Lys Phe His
           35                  40                  45
   cca aac ctc ctt cgt cag att tgt tta tgc ggt tac tat gag aac ctg      192
   Pro Asn Leu Leu Arg Gln Ile Cys Leu Cys Gly Tyr Tyr Glu Asn Leu
       50                  55                  60
   gaa aaa gga atc aca ctg ttt cgc caa ggg gat att gga acc aac tgg      240
   Glu Lys Gly Ile Thr Leu Phe Arg Gln Gly Asp Ile Gly Thr Asn Trp
   65                  70                  75                  80
   tat gct gtc ctg gct ggg tct ttg gat gtt aaa gtg tct gag acc agc      288
   Tyr Ala Val Leu Ala Gly Ser Leu Asp Val Lys Val Ser Glu Thr Ser
                   85                  90                  95
   agt cac cag gat gcg gtg acc atc tgc act ctg gga att ggg aca gcc      336
   Ser His Gln Asp Ala Val Thr Ile Cys Thr Leu Gly Ile Gly Thr Ala
               100                 105                 110
   ttt gaa gag tcc att ctg gat aac acc cct cgc cat gca acc atc gtt      384
   Phe Glu Glu Ser Ile Leu Asp Asn Thr Pro Arg His Ala Thr Ile Val
           115                 120                 125
   acc agg gag agc agc gaa ctt ctc cgc att gag cag gag gac ttc aag        432
   Thr Arg Glu Ser Ser Glu Leu Leu Arg Ile Glu Gln Glu Asp Phe Lys
       130                 135                 140
   gca cta tgg gag aaa tac cga cag tat atg gcc gga ctt ctg gct cct        480
   Ala Leu Trp Glu Lys Tyr Arg Gln Tyr Met Ala Gly Leu Leu Ala Pro
   145                 150                 155                 160
   ccc tat ggt gtt atg gaa acg ggc tct aac aat gac agg att cct gac        528
   Pro Tyr Gly Val Met Glu Thr Gly Ser Asn Asn Asp Arg Ile Pro Asp
                   165                 170                 175
   aag gag aat aca cct ctc att gaa ccc cac gtt cct ctc cgt cct gct        576
   Lys Glu Asn Thr Pro Leu Ile Glu Pro His Val Pro Leu Arg Pro Ala
               180                 185                 190
   cac acc att acc aag gtc cct tca gag aag atc ctc aga gct gga aaa        624
   His Thr Ile Thr Lys Val Pro Ser Glu Lys Ile Leu Arg Ala Gly Lys
           195                 200                 205
   att tta cga att gcc att ctc tct cga gct ccc cac atg ata aga gac        672
   Ile Leu Arg Ile Ala Ile Leu Ser Arg Ala Pro His Met Ile Arg Asp
       210                 215                 220
   aga aag tac cac cta aag aca tac aga caa tgc tgt gtt ggg act gag        720
   Arg Lys Tyr His Leu Lys Thr Tyr Arg Gln Cys Cys Val Gly Thr Glu
   225                 230                 235                 240
   ctg gta gac tgg atg ata cag cag aca tcc tgt gtt cac tcg cgg act        768
   Leu Val Asp Trp Met Ile Gln Gln Thr Ser Cys Val His Ser Arg Thr
                   245                 250                 255
   caa gct gtt ggc atg tgg caa gtc ttg ctg gaa gat ggt gtc ctc aac        816
   Gln Ala Val Gly Met Trp Gln Val Leu Leu Glu Asp Gly Val Leu Asn
               260                 265                 270
   cat gtg gac cag gag cgc cat ttc caa gac aaa tat tta ttt tat cga        864
   His Val Asp Gln Glu Arg His Phe Gln Asp Lys Tyr Leu Phe Tyr Arg
           275                 280                 285
   ttt ctg gat gac gag cgt gag gat gcc cct ttg cct act gag gaa gag        912
   Phe Leu Asp Asp Glu Arg Glu Asp Ala Pro Leu Pro Thr Glu Glu Glu
       290                 295                 300
   aag aag gag tgt gat gaa gaa ctt cag gac acc atg ctg ctg ctc tca        960
   Lys Lys Glu Cys Asp Glu Glu Leu Gln Asp Thr Met Leu Leu Leu Ser
   305                 310                 315                 320
   cag atg ggc cct gac gcc cac atg aga atg atc ctg cga aaa cca cct       1008
   Gln Met Gly Pro Asp Ala His Met Arg Met Ile Leu Arg Lys Pro Pro
                   325                 330                 335
   ggc cag agg act gtg gat gac cta gag att atc tac gac gag ctc ctt       1056
   Gly Gln Arg Thr Val Asp Asp Leu Glu Ile Ile Tyr Asp Glu Leu Leu
               340                 345                 350
   cat att aaa gcc tta tcc cat ctc tct acc aca gtg aaa cgg gag tta       1104
   His Ile Lys Ala Leu Ser His Leu Ser Thr Thr Val Lys Arg Glu Leu
           355                 360                 365
   gca ggt gtt ctc att ttt gag tct cac gcc aaa gga gga act gtg ttg       1152
   Ala Gly Val Leu Ile Phe Glu Ser His Ala Lys Gly Gly Thr Val Leu
       370                 375                 380
   ttt aac cag ggg gaa gaa ggt acc tcc tgg tac atc att ctg aaa gga      1200
   Phe Asn Gln Gly Glu Glu Gly Thr Ser Trp Tyr Ile Ile Leu Lys Gly
   385                 390                 395                 400
   tcc gtg aat gta gtc att tat ggc aag ggt gtg gtc tgc acc ctg cac      1248
   Ser Val Asn Val Val Ile Tyr Gly Lys Gly Val Val Cys Thr Leu His
                   405                 410                 415
   gaa gga gat gac ttt gac aag tta gct cta gtg aac gat gct cca aga      1296
   Glu Gly Asp Asp Phe Asp Lys Leu Ala Leu Val Asn Asp Ala Pro Arg
               420                 425                 430
   gct gcc tcc att gtt ctt cgg gaa gat aat tgt cac ttc cta aga gtc      1344
   Ala Ala Ser Ile Val Leu Arg Glu Asp Asn Cys His Phe Leu Arg Val
           435                 440                 445
   gac aag gaa gac ttc aat cgg att ctg agg gac gtt gag gcg aat aca      1392
   Asp Lys Glu Asp Phe Asn Arg Ile Leu Arg Asp Val Glu Ala Asn Thr
       450                 455                 460
   gtc aga ctt aaa gaa cat gac caa gat gtc ttg gta ctg gag aag gtc      1440
   Val Arg Leu Lys Glu His Asp Gln Asp Val Leu Val Leu Glu Lys Val
   465                 470                 475                 480
   cca gca ggg aac aga gct gct aat caa gga aac tca cag cct cag caa      1488
   Pro Ala Gly Asn Arg Ala Ala Asn Gln Gly Asn Ser Gln Pro Gln Gln
                   485                 490                 495
   aag tat act gtg atg tca gga aca cct gaa aag att tta gag cat ttt      1536
   Lys Tyr Thr Val Met Ser Gly Thr Pro Glu Lys Ile Leu Glu His Phe
               500                 505                 510
   cta gaa aca ata cgc ctt gag cca tcg ttg aat gaa gca aca gat tcg      1584
   Leu Glu Thr Ile Arg Leu Glu Pro Ser Leu Asn Glu Ala Thr Asp Ser
           515                 520                 525
   gtt tta aat gac ttt gtt atg atg cac tgt gtt ttt atg cca aat acc      1632
   Val Leu Asn Asp Phe Val Met Met His Cys Val Phe Met Pro Asn Thr
       530                 535                 540
   cag ctt tgc cct gcc ctt gtg gcc cat tac cac gca cag cct tct caa      1680
   Gln Leu Cys Pro Ala Leu Val Ala His Tyr His Ala Gln Pro Ser Gln
   545                 550                 555                 560
   ggt acc gag cag gag aga atg gat tat gcc ctc aac aac aag agg cgg      1728
   Gly Thr Glu Gln Glu Arg Met Asp Tyr Ala Leu Asn Asn Lys Arg Arg
                   565                 570                 575
   gtc atc cgc ttg gtc ctg cag tgg gcg gcc atg tat ggc gat ctc ctc      1776
   Val Ile Arg Leu Val Leu Gln Trp Ala Ala Met Tyr Gly Asp Leu Leu
               580                 585                 590
   caa gaa gat gat gtg gcc atg gcc ttc ctg gag gag ttc tat gtg tct      1824
   Gln Glu Asp Asp Val Ala Met Ala Phe Leu Glu Glu Phe Tyr Val Ser
           595                 600                 605
   gta tca gat gac gca cgg atg atg gct gcc ttc aag gag cag ctg cca      1872
   Val Ser Asp Asp Ala Arg Met Met Ala Ala Phe Lys Glu Gln Leu Pro
       610                 615                 620
   gag ctg gag aag att gtc aag caa atc tca gaa gac gca aaa gct cca      1920
   Glu Leu Glu Lys Ile Val Lys Gln Ile Ser Glu Asp Ala Lys Ala Pro
   625                 630                 635                 640
   cag aag aag cac aag gtg ctt ttg caa cag ttc aac aca ggt gac gag      1968
   Gln Lys Lys His Lys Val Leu Leu Gln Gln Phe Asn Thr Gly Asp Glu
                   645                 650                 655
   agg gcc cag aag cgt cag cct att cgt ggc tct gat gag gtt ttg ttc      2016
   Arg Ala Gln Lys Arg Gln Pro Ile Arg Gly Ser Asp Glu Val Leu Phe
               660                 665                 670
   aag gtc tac tgc atc gac cac acc tat act acc att cgt gtg ccg gta      2064
   Lys Val Tyr Cys Ile Asp His Thr Tyr Thr Thr Ile Arg Val Pro Val
           675                 680                 685
   gct gcc tcg gtg aag gaa gtc atc agt gca gta gct gac aaa ctg ggc      2112
   Ala Ala Ser Val Lys Glu Val Ile Ser Ala Val Ala Asp Lys Leu Gly
       690                 695                 700
   tca ggg gaa ggc ctg atc atc gtc aag atg aac tct gga gga gaa aag      2160
   Ser Gly Glu Gly Leu Ile Ile Val Lys Met Asn Ser Gly Gly Glu Lys
   705                 710                 715                 720
   gtg gtg ctg aaa tct aat gat gtt tca gta ttt acg acg ctc acc att      2208
   Val Val Leu Lys Ser Asn Asp Val Ser Val Phe Thr Thr Leu Thr Ile
                   725                 730                 735
   aat gga cgc ctg ttt gcc tgc ccg aga gag caa ttc gac tca ctg act      2256
   Asn Gly Arg Leu Phe Ala Cys Pro Arg Glu Gln Phe Asp Ser Leu Thr
               740                 745                 750
   ccc ttg ccg gaa cag gaa ggc ccg acc act ggg aca gtg gga aca ttt      2304
   Pro Leu Pro Glu Gln Glu Gly Pro Thr Thr Gly Thr Val Gly Thr Phe
           755                 760                 765
   gag ctg atg agc tcg aaa gac ctg gcg tac cag atg aca acc tac gat      2352
   Glu Leu Met Ser Ser Lys Asp Leu Ala Tyr Gln Met Thr Thr Tyr Asp
       770                 775                 780
   tgg gaa ctc ttc aac tgt gtg cat gag ctg gag cta atc tac cac aca      2400
   Trp Glu Leu Phe Asn Cys Val His Glu Leu Glu Leu Ile Tyr His Thr
   785                 790                 795                 800
   ttt gga agg cat aat ttt aaa aag acc acg gca aac ttg gat ttg ttc      2448
   Phe Gly Arg His Asn Phe Lys Lys Thr Thr Ala Asn Leu Asp Leu Phe
                   805                 810                 815
   ctg agg agg ttt aat gaa att cag ttt tgg gtt gtc act gag gtc tgc      2496
   Leu Arg Arg Phe Asn Glu Ile Gln Phe Trp Val Val Thr Glu Val Cys
               820                 825                 830
   ctt tgt tcc cag ctc agc aaa cgt gtt cag ctt ttg aaa aaa ttt atc      2544
   Leu Cys Ser Gln Leu Ser Lys Arg Val Gln Leu Leu Lys Lys Phe Ile
           835                 840                 845
   aag ata gcg gct cac tgc aag gag tac aaa aat cta aat tcc ttt ttc      2592
   Lys Ile Ala Ala His Cys Lys Glu Tyr Lys Asn Leu Asn Ser Phe Phe
       850                 855                 860
   gcc atc gtc atg gga ctc agc aac gtg gcc gtg agc cgc ttg gca cta      2640
   Ala Ile Val Met Gly Leu Ser Asn Val Ala Val Ser Arg Leu Ala Leu
   865                 870                 875                 880
   acg tgg gag aaa ctg ccg agc aag ttt aag aag ttc tat gcg gag ttt      2688
   Thr Trp Glu Lys Leu Pro Ser Lys Phe Lys Lys Phe Tyr Ala Glu Phe
                   885                 890                 895
   gag agc ttg atg gat cct tcc aga aac cac agg gca tac agg ctg aca      2736
   Glu Ser Leu Met Asp Pro Ser Arg Asn His Arg Ala Tyr Arg Leu Thr
           900                     905                     910
   gca gcc aag ctg gag ccc cct ctc atc cct ttc atg ccc ttg ctt att         2784
   Ala Ala Lys Leu Glu Pro Pro Leu Ile Pro Phe Met Pro Leu Leu Ile
               915                     920                     925
   aaa gat atg aca ttt act cat gag ggg aac aag acg ttc att gac aat         2832
   Lys Asp Met Thr Phe Thr His Glu Gly Asn Lys Thr Phe Ile Asp Asn
       930                     935                     940
   cta gta aac ttt gaa aaa atg cgc atg att gca aac act gcc aga aca         2880
   Leu Val Asn Phe Glu Lys Met Arg Met Ile Ala Asn Thr Ala Arg Thr
   945                     950                     955                     960
   gta cgg tac tac agg agc cag ccc ttc aat ccg gat gcc gct caa gct         2928
   Val Arg Tyr Tyr Arg Ser Gln Pro Phe Asn Pro Asp Ala Ala Gln Ala
                   965                     970                     975
   aat aag aac cat cag gat gtc cgg agt tat gta cgg caa tta aat gtg         2976
   Asn Lys Asn His Gln Asp Val Arg Ser Tyr Val Arg Gln Leu Asn Val
               980                     985                     990
   att gac aac cag aga act tta tca  cag atg tca cac aga  tta gag cct       3024
   Ile Asp Asn Gln Arg Thr Leu Ser  Gln Met Ser His Arg  Leu Glu Pro
           995                     1000                    1005
   cga agg  cca tag                                                        3036
   Arg Arg  Pro
       1010
<210> 8
   <211> 1011
   <212> PRT
   <213> Mus musculus
<400> 8
   Met Val Ala Ala His Ala Ala His Ser Gln Ser Ser Ala Glu Trp Ile
   1               5                   10                  15
   Ala Cys Leu Asp Lys Arg Pro Leu Glu Arg Ser Ser Glu Asp Val Asp
               20                  25                  30
   Ile Ile Phe Thr Arg Leu Lys Gly Val Lys Ala Phe Glu Lys Phe His
           35                  40                  45
   Pro Asn Leu Leu Arg Gln Ile Cys Leu Cys Gly Tyr Tyr Glu Asn Leu
       50                  55                  60
   Glu Lys Gly Ile Thr Leu Phe Arg Gln Gly Asp Ile Gly Thr Asn Trp
   65                  70                  75                  80
   Tyr Ala Val Leu Ala Gly Ser Leu Asp Val Lys Val Ser Glu Thr Ser
                   85                  90                  95
   Ser His Gln Asp Ala Val Thr Ile Cys Thr Leu Gly Ile Gly Thr Ala
               100                 105                 110
   Phe Glu Glu Ser Ile Leu Asp Asn Thr Pro Arg His Ala Thr Ile Val
           115                 120                 125
   Thr Arg Glu Ser Ser Glu Leu Leu Arg Ile Glu Gln Glu Asp Phe Lys
       130                 135                 140
   Ala Leu Trp Glu Lys Tyr Arg Gln Tyr Met Ala Gly Leu Leu Ala Pro
   145                 150                 155                 160
   Pro Tyr Gly Val Met Glu Thr Gly Ser Asn Asn Asp Arg Ile Pro Asp
                   165                 170                 175
   Lys Glu Asn Thr Pro Leu Ile Glu Pro His Val Pro Leu Arg Pro Ala
               180                 185                 190
   His Thr Ile Thr Lys Val Pro Ser Glu Lys Ile Leu Arg Ala Gly Lys
           195                 200                 205
   Ile Leu Arg Ile Ala Ile Leu Ser Arg Ala Pro His Met Ile Arg Asp
       210                 215                 220
   Arg Lys Tyr His Leu Lys Thr Tyr Arg Gln Cys Cys Val Gly Thr Glu
   225                 230                 235                 240
   Leu Val Asp Trp Met Ile Gln Gln Thr Ser Cys Val His Ser Arg Thr
                   245                 250                 255
   Gln Ala Val Gly Met Trp Gln Val Leu Leu Glu Asp Gly Val Leu Asn
               260                 265                 270
   His Val Asp Gln Glu Arg His Phe Gln Asp Lys Tyr Leu Phe Tyr Arg
           275                 280                 285
   Phe Leu Asp Asp Glu Arg Glu Asp Ala Pro Leu Pro Thr Glu Glu Glu
       290                 295                 300
   Lys Lys Glu Cys Asp Glu Glu Leu Gln Asp Thr Met Leu Leu Leu Ser
   305                 310                 315                 320
   Gln Met Gly Pro Asp Ala His Met Arg Met Ile Leu Arg Lys Pro Pro
                   325                 330                 335
   Gly Gln Arg Thr Val Asp Asp Leu Glu Ile Ile Tyr Asp Glu Leu Leu
               340                 345                 350
   His Ile Lys Ala Leu Ser His Leu Ser Thr Thr Val Lys Arg Glu Leu
           355                 360                 365
   Ala Gly Val Leu Ile Phe Glu Ser His Ala Lys Gly Gly Thr Val Leu
   370 375 380
   Phe Asn Gln Gly Glu Glu Gly Thr Ser Trp Tyr Ile Ile Leu Lys Gly
   385 390 395 400
   Ser Val Asn Val Val Ile Tyr Gly Lys Gly Val Val Cys Thr Leu His
   405 410 415
   Glu Gly Asp Asp Phe Asp Lys Leu Ala Leu Val Asn Asp Ala Pro Arg
   420 425 430
   Ala Ala Ser Ile Val Leu Arg Glu Asp Asn Cys His Phe Leu Arg Val
   435 440 445
   Asp Lys Glu Asp Phe Asn Arg Ile Leu Arg Asp Val Glu Ala Asn Thr
   450 455 460
   Val Arg Leu Lys Glu His Asp Gln Asp Val Leu Val Leu Glu Lys Val
   465 470 475 480
   Pro Ala Gly Asn Arg Ala Ala Asn Gln Gly Asn Ser Gln Pro Gln Gln
   485 490 495
   Lys Tyr Thr Val Met Ser Gly Thr Pro Glu Lys Ile Leu Glu His Phe
   500 505 510
   Leu Glu Thr Ile Arg Leu Glu Pro Ser Leu Asn Glu Ala Thr Asp Ser
   515 520 525
   Val Leu Asn Asp Phe Val Met Met His Cys Val Phe Met Pro Asn Thr
   530 535 540
   Gln Leu Cys Pro Ala Leu Val Ala His Tyr His Ala Gln Pro Ser Gln
   545 550 555 560
   Gly Thr Glu Gln Glu Arg Met Asp Tyr Ala Leu Asn Asn Lys Arg Arg
   565 570 575
   Val Ile Arg Leu Val Leu Gln Trp Ala Ala Met Tyr Gly Asp Leu Leu
   580 585 590
   Gln Glu Asp Asp Val Ala Met Ala Phe Leu Glu Glu Phe Tyr Val Ser
   595 600 605
   Val Ser Asp Asp Ala Arg Met Met Ala Ala Phe Lys Glu Gln Leu Pro
   610 615 620
   Glu Leu Glu Lys Ile Val Lys Gln Ile Ser Glu Asp Ala Lys Ala Pro
   625 630 635 640
   Gln Lys Lys His Lys Val Leu Leu Gln Gln Phe Asn Thr Gly Asp Glu
   645 650 655
   Arg Ala Gln Lys Arg Gln Pro Ile Arg Gly Ser Asp Glu Val Leu Phe
   660 665 670
   Lys Val Tyr Cys Ile Asp His Thr Tyr Thr Thr Ile Arg Val Pro Val
   675 680 685
   Ala Ala Ser Val Lys Glu Val Ile Ser Ala Val Ala Asp Lys Leu Gly
   690 695 700
   Ser Gly Glu Gly Leu Ile Ile Val Lys Met Asn Ser Gly Gly Glu Lys
   705 710 715 720
   Val Val Leu Lys Ser Asn Asp Val Ser Val Phe Thr Thr Leu Thr Ile
   725 730 735
   Asn Gly Arg Leu Phe Ala Cys Pro Arg Glu Gln Phe Asp Ser Leu Thr
   740 745 750
   Pro Leu Pro Glu Gln Glu Gly Pro Thr Thr Gly Thr Val Gly Thr Phe
   755 760 765
   Glu Leu Met Ser Ser Lys Asp Leu Ala Tyr Gln Met Thr Thr Tyr Asp
   770 775 780
   Trp Glu Leu Phe Asn Cys Val His Glu Leu Glu Leu Ile Tyr His Thr
   785 790 795 800
   Phe Gly Arg His Asn Phe Lys Lys Thr Thr Ala Asn Leu Asp Leu Phe
   805 810 815
   Leu Arg Arg Phe Asn Glu Ile Gln Phe Trp Val Val Thr Glu Val Cys
   820 825 830
   Leu Cys Ser Gln Leu Ser Lys Arg Val Gln Leu Leu Lys Lys Phe Ile
   835 840 845
   Lys Ile Ala Ala His Cys Lys Glu Tyr Lys Asn Leu Asn Ser Phe Phe
   850 855 860
   Ala Ile Val Met Gly Leu Ser Asn Val Ala Val Ser Arg Leu Ala Leu
   865 870 875 880
   Thr Trp Glu Lys Leu Pro Ser Lys Phe Lys Lys Phe Tyr Ala Glu Phe
   885 890 895
   Glu Ser Leu Met Asp Pro Ser Arg Asn His Arg Ala Tyr Arg Leu Thr
   900 905 910
   Ala Ala Lys Leu Glu Pro Pro Leu Ile Pro Phe Met Pro Leu Leu Ile
   915 920 925
   Lys Asp Met Thr Phe Thr His Glu Gly Asn Lys Thr Phe Ile Asp Asn
   930 935 940
   Leu Val Asn Phe Glu Lys Met Arg Met Ile Ala Asn Thr Ala Arg Thr
   945 950 955 960
   Val Arg Tyr Tyr Arg Ser Gln Pro Phe Asn Pro Asp Ala Ala Gln Ala
   965 970 975
   Asn Lys Asn His Gln Asp Val Arg Ser Tyr Val Arg Gln Leu Asn Val
   980 985 990
   Ile Asp Asn Gln Arg Thr Leu Ser Gln Met Ser His Arg Leu Glu Pro
   995 1000 1005
   Arg Arg Pro
   1010
<210> 9
   <211> 20
   <212> DNA
   <213> Artificial
<220>
   <223> Primer BglII-Epac2
<400> 9
   agatctatgg tcgctgcgca 20
<210> 10
   <211> 18
   <212> DNA
   <213> Artificial
<220>
   <223> Primer Epac2-EcoRI
<400> 10
   gaattctggc cttcgagg 18
<210> 11
   <211> 94
   <212> DNA
   <213> Artificial
<220>
   <223> Multicloning site, pECFP-C1
<400> 11
   tacaagtccg gactcagatc tcgagctcaa gcttcgaatt ctgcagtcga cggtaccgcg 60
   ggcccgggat ccaccggatc tagataactg atca 94
<210> 12
   <211> 720
   <212> DNA
   <213> Artificial
<220>
   <223> ECFP
<220>
   <221> CDS
   <222> (1) .. (720)
<400> 12
   atg gtg agc aag ggc gag gag ctg ttc acc ggg gtg gtg ccc atc ctg      48
   Met Val Ser Lys Gly Glu Glu Leu Phe Thr Gly Val Val Pro Ile Leu
   1               5                   10                  15
   gtc gag ctg gac ggc gac gta aac ggc cac aag ttc agc gtg tcc ggc      96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc     144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ctg acc tgg ggc gtg cag tgc ttc agc cgc tac ccc gac cac atg aag     240
   Leu Thr Trp Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac atc agc cac aac gtc tat atc acc gcc gac aag cag aag aac     480
   Asn Tyr Ile Ser His Asn Val Tyr Ile Thr Ala Asp Lys Gln Lys Asn
   145                 150                 155                 160
   ggc atc aag gcc aac ttc aag atc cgc cac aac atc gag gac ggc agc     528
   Gly Ile Lys Ala Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag tga     720
   Val Thr Ala Ala Gly Ile Thr Leu Gly Met Asp Glu Leu Tyr Lys
   225                 230                 235
<210> 13
   <211> 239
   <212> PRT
   <213> Artificial
<220>
   <223> Synthetic Construct
<400> 13
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Trp Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Ile Ser His Asn Val Tyr Ile Thr Ala Asp Lys Gln Lys Asn
   145                 150                 155                 160
   Gly Ile Lys Ala Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 14
   <211> 94
   <212> DNA
   <213> Artificial
<220>
   <223> Multicloning site, pEYFP-N1
<400> 14
   gctagcgcta ccggactcag atctcgagct caagcttcga attctgcagt cgacggtacc        60
   gcgggcccgg gatccaccgg tcgccaccat ggtg                                    94
<210> 15
   <211> 16
   <212> DNA
   <213> Artificial
<220>
   <223> Primer GFP-fw
<400> 15
   atggtgagca agggcg 16
<210> 16
   <211> 18
   <212> DNA
   <213> Artificial
<220>
   <223> Primer GFP-rv
<400> 16
   cttgtacagc tcgtccat 18
<210> 17
   <211> 19
   <212> DNA
   <213> Artificial
<220>
   <223> Primer EcoRI-EYFP
<400> 17
   gaattcatgg tgagcaagg 19
<210> 18
   <211> 20
   <212> DNA
   <213> Artificial
<220>
   <223> Primer EYFP-EcoRI
<400> 18
   gaattccttg tacagctcgt 20
<210> 19
   <211> 720
   <212> DNA
   <213> Artificial
<220>
   <223> EYFP
<220>
   <221> CDS
   <222> (1)..(720)
<400> 19
   atg gtg agc aag ggc gag gag ctg ttc acc ggg gtg gtg ccc atc ctg        48
   Met Val Ser Lys Gly Glu Glu Leu Phe Thr Gly Val Val Pro Ile Leu
   1               5                   10                  15
   gtc gag ctg gac ggc gac gta aac ggc cac aag ttc agc gtg tcc ggc        96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc       144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc       192
   Cys Thr Thr Gly Lys Leu Pro Val Pro Trp Pro Thr Leu Val Thr Thr
       50                  55                  60
   ttc ggc tac ggc ctg cag tgc ttc gcc cgc tac ccc gac cac atg aag       240
   Phe Gly Tyr Gly Leu Gln Cys Phe Ala Arg Tyr Pro Asp His Met Lys
   65                  70                  75                  80
   cag cac gac ttc ttc aag tcc gcc atg ccc gaa ggc tac gtc cag gag       288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag       336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc       384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac       432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac       480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc       528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc       576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg       624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc       672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag tga       720
   Val Thr Ala Ala Gly Ile Thr Leu Gly Met Asp Glu Leu Tyr Lys
   225                 230                 235
<210> 20
   <211> 239
   <212> PRT
   <213> Artificial
<220>
   <223> Synthetic Construct
<400> 20
   Met Val Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Phe Gly Tyr Gly Leu Gln Cys Phe Ala Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235